# EUROPEAN PATENT SPECIFICATION

(11) **EP 4 076 499 B1**
(45) Date of publication and mention of the grant of the patent: **16.07.2025**
(21) Application number: 20901160.0
(22) Date of filing: 18.12.2020
(51) Int. Cl.: A61K 38/17, A61K 9/10, A61P 25/02, A61K 9/00, A61K 38/18, A61P 41/00

(54) **USE OF GLIAL CELL LINE-DERIVED NEUROTROPHIC FACTOR (GDNF) FOR THE TREATMENT OF ENTERIC NEUROPATHIES**
VERWENDUNG VON AUS GLIALZELLEN ABGELEITETEN NEUROTROPHEN FAKTOREN (GDNF) FÜR DIE BEHANDLUNG VON ENTERISCHEN NEUROPATHIEN
UTILISATION DU FACTEUR NEUROTROPHIQUE DÉRIVÉ DES CELLULES GLIALES (GDNF) POUR LE TRAITEMENT DE NEUROPATHIES ENTÉRIQUES

(30) Priority: 19.12.2019 US 201962950781 P
(43) Date of publication of application: 26.10.2022
(73) Proprietor: Transfert Plus Societe en Commandite, Québec, Québec G1P 4P5 (CA)
(72) Inventor: SORET, Rodolphe, Montréal, Québec H2J 2N1 (CA); PILON, Nicolas, Montréal, Québec H2J 1R9 (CA)
(74) Representative: Plasseraud IP
(86) International application number: PCT/CA2020/051746
(87) International publication number: WO 2021/119827

(56) References cited:
- WO-A1-2012/141936
- WO-A1-97/11964
- SORET R. ET AL: "A molecular-based regenerative medicine approach for the treatment of hirschsprung disease", NEUROGASTROENTEROLOGY AND MOTILITY, vol. 31, 1 August 2019 (2019-08-01), GB, XP093078168, ISSN: 1350-1925, Retrieved from the Internet <URL:https://onlinelibrary.wiley.com/doi/full-xml/10.1111/nmo.13671> DOI: 10.1111/nmo.13671
- SORET R. ET AL: "A molecular-based regenerative medicine approach for the treatment of hirschsprung disease", NEUROGASTROENTEROLOGY AND MOTILITY, vol. 31, 1 August 2019 (2019-08-01), GB, XP093078168, ISSN: 1350-1925, Retrieved from the Internet <URL:https://onlinelibrary.wiley.com/doi/full-xml/10.1111/nmo.13671> DOI: 10.1111/nmo.13671
- SORET, RODOLPHE; SCHNEIDER SABINE; BERNAS GUILLAUME; CHRISTOPHERS BRIANA; SOUCHKOVA OULIANA; CHARRIER BAPTISTE; RIGHINI-GRUNDER FR: "Glial Cell -Derived Neurotrophic Factor Induces Enteric Neurogenesis and Improves Colon Structure and Function in Mouse Models of Hirsch sprung Disease", GASTROENTEROLOGY, vol. 159, no. 5, November 2020 (2020-11-01), pages 1824 - 1838, XP086348979, ISSN: 0016-5085, DOI: 10.1053/j.gastro.2020.07.018
- BONDURAND, N ET AL.: "Mouse models of Hirschsprung disease and other developmental disorders of the enteric nervous system: Old and new players", DEVELOPMENTAL BIOLOGY, vol. 417, 15 September 2016 (2016-09-15), pages 139 - 157, XP029729376, ISSN: 0012- 1606, DOI: 10.1016/j.ydbio.2016.06.042
- MCKEOWN, SONJA J., MOHSENIPOUR MITRA, BERGNER ANNETTE J., YOUNG HEATHER M., STAMP LINCON A.: "Exposure to GDNF Enhances the Ability of Enteric Neural Progenitors to Generate an Enteric Nervous System", STEM CELL REPORTS, vol. 8, no. 2, 14 February 2017 (2017-02-14), pages 476 - 488, XP055836392, ISSN: 2213-6711, DOI: 10.1016/j.stemcr.2016.12.013
- TOMAC, A. ET AL.: "Protection and repair of the nigrostriatal dopaminergic system by GDNF in vivo", NATURE, vol. 373, no. 6512, 26 January 1995 (1995-01-26), pages 335 - 339, XP037115429, ISSN: 0028-0836, DOI: 10.1038/373335a0

## Description

### TECHNICAL FIELD

The present invention generally relates to the treatment of enteric neuropathies such as Hirschsprung disease (HSCR) and intestinal hypoganglionosis.

### BACKGROUND ART

The enteric nervous system (ENS) extends along the entire gastrointestinal tract to control bowel motility, blood flow and epithelial activity in response to sensory stimuli (1). Interconnected enteric ganglia containing neurons and glia develop from neural crest-derived progenitors that migrate through the intestine during prenatal development. Incomplete colonization of distal colon by ENS progenitors causes Hirschsprung disease (HSCR), a condition affecting 1 in 5000 newborns (2,3). In HSCR, distal colon without neural ganglia (i.e., aganglionic colon) remains tonically contracted and does not propagate contractions, causing functional intestinal obstruction. HSCR symptoms include refractory constipation with retention of stool and air, abdominal distension, growth failure, occasional vomiting, bowel inflammation (enterocolitis) and a risk of bacterial translocation into blood causing sepsis and premature death (2).

HSCR is clinically subdivided into short-segment (S-HSCR) and long-segment forms (L-HSCR) (4). S-HSCR, which occurs in >80% of cases, means the ENS is absent from rectum and sigmoid colon. L-HSCR means longer regions of distal bowel are aganglionic. HSCR etiology remains incompletely understood, but many genes influence HSCR risk (2). Furthermore, genetic risk variants may combine with non-genetic factors to prevent full bowel colonization by ENS progenitors (5). This non-Mendelian inheritance occurs because many proteins must work together for normal ENS development.

Since 1948, most children with HSCR have had their life saved by surgical removal of distal aganglionic bowel (16, 17). However, this procedure is far from ideal. Post-surgical complications are common and can also be long-lasting, impacting survival (e.g. enterocolitis) and/or quality of life (*e.g.,* fecal incontinence or obstructive symptoms) (18-20). One promising approach would be "regenerative medicine" to rebuild the ENS and reduce the need for surgery. This idea prompted many groups to develop cell transplantation-based HSCR therapies (21). However, despite many encouraging results, some difficulties remain (22). The optimal source of stem cells, ideal amplification and/or differentiation strategies prior to transplantation, methods of cell delivery, and cell function and fate after transplantation are not yet well defined. Additionally, non-autonomous cell transplantation may require immunosuppression.

Hypoganglionosis, also known as intestinal hypoganglionosis, is a disorder causing a reduced number of nerves in the intestinal wall. Intestinal hypoganglionosis can mimic HSCR; patients with both conditions may present with chronic constipation, intestinal obstruction, and enterocolitis (inflammation of the intestines). Patients with hypoganglionosis may also suffer from severe complications including fecaloma (hardening of the feces inside the colon), bleeding or perforation of the intestine, and breathing problems resulting from a distended colon. The exact cause of hypoganglionosis is often not known. In some cases, it is due to factors present at birth (congenital), while other times it is believed to be an acquired condition. The management of isolated hypoganglionosis generally involves surgery to remove the affected bowel segment.

There is thus clearly a need for alternative treatments for enteric neuropathies such as HSCR and intestinal hypoganglionosis, notably treatments aimed at inducing neurogenesis in the distal colon and restoring distal colon motility in HSCR and intestinal hypoganglionosis patients.

### SUMMARY OF THE INVENTION

The present disclosure relates to a pharmaceutical compositin comprising a recombinant Glial cell line-Derived Neurotrophic Factor (GDNF) polypeptide and a pharmaceutically acceptable carrier for use in treating an enteric neuropathy in a human subject by inducing enteric neurogenesis in an aganglionic or hypoganglionic segment of the distal colon, wherein the composition is administered into the distal colon of the subject rectally via enema at a dose of recombinant GDNF polypeptide of about 10 mg to about 15 mg per kg.

In certain embodiments, the GDNF polypeptide comprises an amino acid sequence having at least 90% identity with amino acids 78-211 of SEQ ID NO: 1.

In certain embodiments, the GDNF polypeptide comprises amino acids 78-211 of SEQ ID NO: 1.

In certain embodiments, the recombinant GDNF polypeptide is at a concentration of 0.5 mg/ml to 2 mg/ml.

In certain embodiments, the pharmaceutically acceptable carrier is a saline solution or a gelling agent.

In certain embodiments, the pharmaceutical composition is administered (i) once-a-day up to four times a day; and/or for at least 2 consecutive days.

In certain embodiments, the pharmaceutical composition is administered prior to or after surgical removel of the aganglionic or hypoanglionic segment in the subject.

In certain embodiments, the enteric neuropathy is intestinal hypoganglionosis.

In certain embodiments, the enteric neuropathy is Hirschsprung disease (HSCR).

In certain embodiments, the subject suffers from short-segment HSCR.

In certain embodiments, the HSCR is sporadic HSCR.

In certain embodiments, the HSCR is associated with a reduced expression or activity of the RET-receptor.

In certain embodiments, the pharmaceutical composition (i) corrects the imbalance of nitrergic and cholinergic neuron subtypes located upstream of the aganglionic or hypoganglionic segment, (ii) restores distal colon motility in the subject, and/or restores the proportions of lymphoid and/or myeloid immune cells in the distal colon of the subject.

In certain embodiments, the human subject is less than 5-year-old.

Other objects, advantages and features of the present invention will become more apparent upon reading of the following non-restrictive description of specific embodiments thereof, given by way of example only with reference to the accompanying drawings.

### BRIEF DESCRIPTION OF DRAWINGS

In the appended drawings:

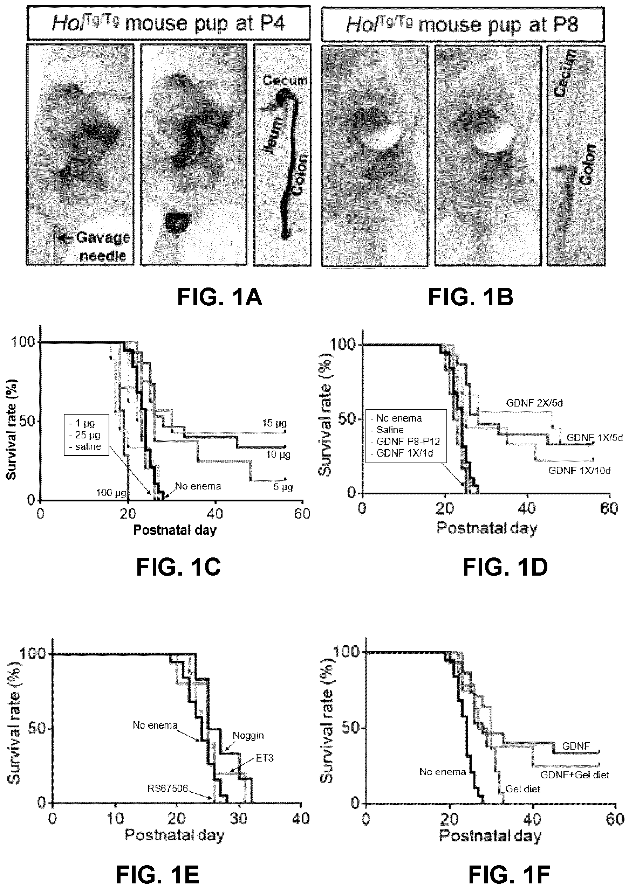

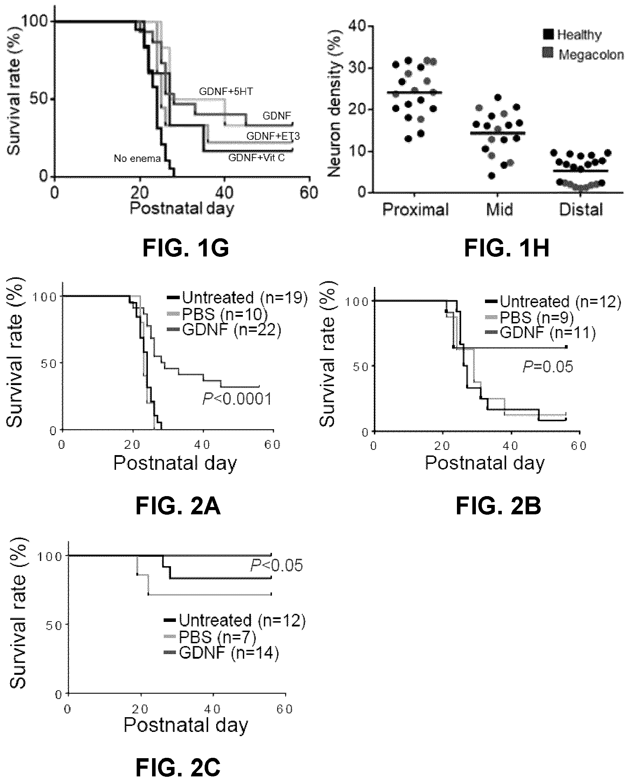

**FIGs. 1A-H**show the set-up of GDNF therapy parameters in *Hol*^{*Tg*/*Tg*} mice. **(****FIG. 1A-B** Distribution of 10µl methylene blue enemas in the colon of P4 **(****FIG. 1A****)** and P8 **(****FIG. 1B****)** *Hol*^{*Tg*/*Tg*} pups. **(****FIG. 1C****)** Impact of GDNF concentration on survival of *Hol*^{*Tg*/*Tg*} pups that received 10µl enemas once daily between P4-P8. Indicated amounts correspond to the total quantity of GDNF that was administered each day. **(****FIG. 1D****)** Impact of treatment time window (P4-P8 vs. P8-P12), duration (1d, 5d or 10d; starting at P4) and frequency (once or twice a day, for 5 days) on the survival of *Hol*^{*Tg*/*Tg*} pups treated with GDNF enemas (quantity of GDNF administered per single enema was kept constant at 10µg in 10µl). (FIG. 1E) Survival rate of *Hol*^{*Tg*/*Tg*} pups that were administered 10µl enemas containing the indicated neurotrophic molecule (Noggin, Endothelin-3, or the serotonin receptor agonist RS67506; all at 1µg/µl final concentration) once daily between P4-P8. **(****FIG. 1F****)** Impact of food consistency (regular chow vs gel diet) on survival of *Hol*^{*Tg*/*Tg*} pups that received GDNF enemas (10µg in 10µl) on a daily basis between P4-P8. **(****FIG. 1G****)** Impact of coadministration of ascorbic acid (Vit.C; 100µM final concentration), serotonin (5-HT; 1µg/µl final concentration) and Endothelin-3 (ET3; 1µg/µl final concentration) on survival of *Hol*^{*Tg*/*Tg*} pups that received GDNF enemas (10µg in 10µl) once daily between P4-P8. **(****FIG. 1H****)** Neuron density in the colon (expressed in % of surface area) and associated health status of P20 *Hol*^{*Tg*/*Tg*} mice that received GDNF enemas (10µg in 10µl) on a daily basis between P4-P8.

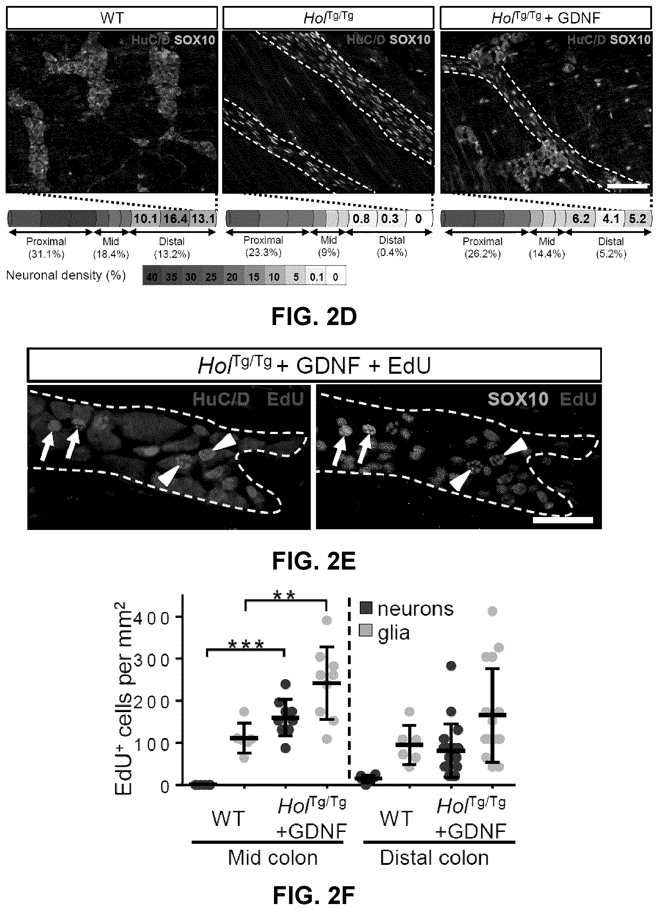

**FIGs. 2A-E**show that GDNF enemas rescue aganglionic megacolon in HSCR mouse models. **(****FIGs. 2A-C**Daily administration of GDNF enemas to *Hol*^{*Tg*/*Tg*} **(****FIG. 2A****),** *Ednrb*^{*S-I*/*s-I*} (FIG. **2B****)** and *TashT*^{*Tg*/*Tg*} **(****FIG. 2C****)** mice between P4-P8 positively impacts both megacolon symptoms (i.e. distal blockage and accumulation of fecal material in the colon, retarded growth, lethargy, and hunched posture) and survival rates (Mantel-Cox statistical test, comparing GDNF-treated and non-treated groups in the case of *Hol*^{*Tg*/*Tg*} and *Ednrb*^{*S-I*/*s-I*}, or GDNF-treated and PBS-treated groups in the case of *TashT*^{*Tg*/*Tg*})*.* **(****FIG. 2D****)** Whole-mount immunofluorescence staining of colonic muscle strips from P20 mice shows that the 5-day GDNF treatment induces myenteric ganglia containing HuC/D⁺ neurons and Sox10⁺ glia in the otherwise aganglionic region of *Hol*^{*Tg*/*Tg*} mice. Dashed outline marks area occupied by extrinsic nerve fibers. Below each representative image is a schematic representation of the average neuronal density for each colon sub-region (represented by cylinders) along the length of the colon. Neuronal density is expressed as the percentage of area occupied by HuC/D⁺ cells in a single confocal slice at the level of the myenteric plexus within the bowel wall (n=6 mice per group; 3 fields of view per sub-region). For each distal colon subregion, the neuronal density is also given as a numerical value. **(****FIG. 2E****)** Immunofluorescence analysis of distal colonic muscularis from P20 mice that received intraperitoneal injections of EdU during the 5-day GDNF treatment demonstrates that a subset of induced myenteric neurons (arrowheads) and glia (arrows) were generated from a dividing precursor during the treatment period. Dashed outline marks area occupied by a single ganglion. **(****FIG. 2F****)** Quantification of EdU incorporation in myenteric neurons and glia in the distal colon. Results are expressed as the number of EdU⁺ cells per mm² (n=3 WT and 3 GDNF-treated *Hol*^{*Tg*/*Tg*} mice; 2-7 fields of view per animal; ****P<0.001; ****P<0.0001;* one-way ANOVA with post-hoc Sidak's test). All displayed images represent a z-stack projection at the level of the myenteric plexus. Scale bars, 100 µm **(****FIG. 2D****)** and 50 µm **(****FIG. 2E****).**

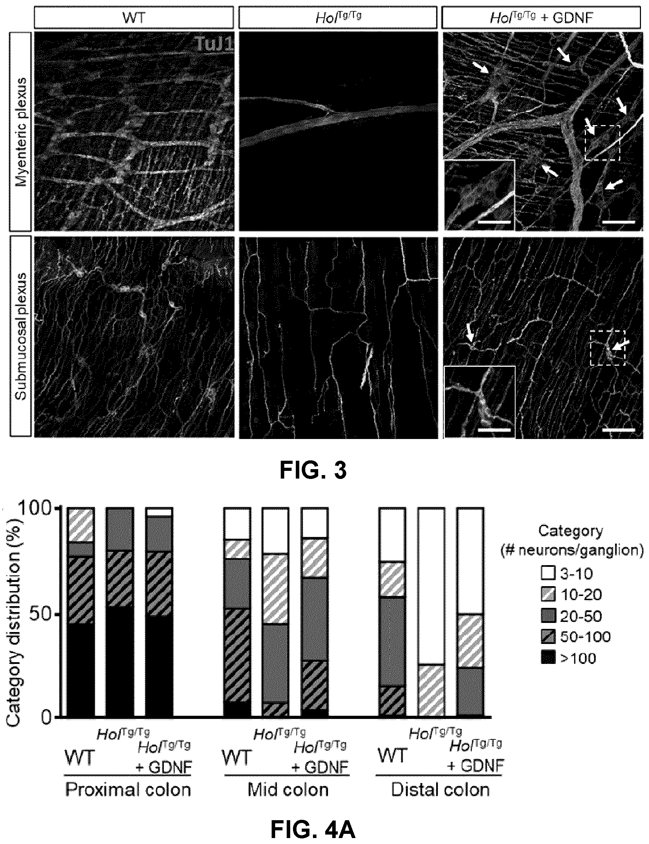

**FIG. 3** shows an overview of myenteric plexus and submucosal plexuses in the distal colon of WT, untreated *Hol*^{*Tg*/*Tg*} or GDNF-treated *Hol*^{*Tg*/*Tg*} mice at P20. Immunofluorescence analysis of TuJ1⁺ neuronal structures in myenteric (upper panels) and submucosal (lower panels) plexus. Arrows point to GDNF-induced ganglia that contain neuronal bodies. Insets are zoomed-in views of GDNF-induced ganglia in dashed boxes. GDNF-treated mice received 10µg GDNF in 10µL enemas once daily from P4-P8. All images show a z-stack projection representative of observations made from 3 mice. Scale bar, 100µm (large panels) and 50µm (insets).

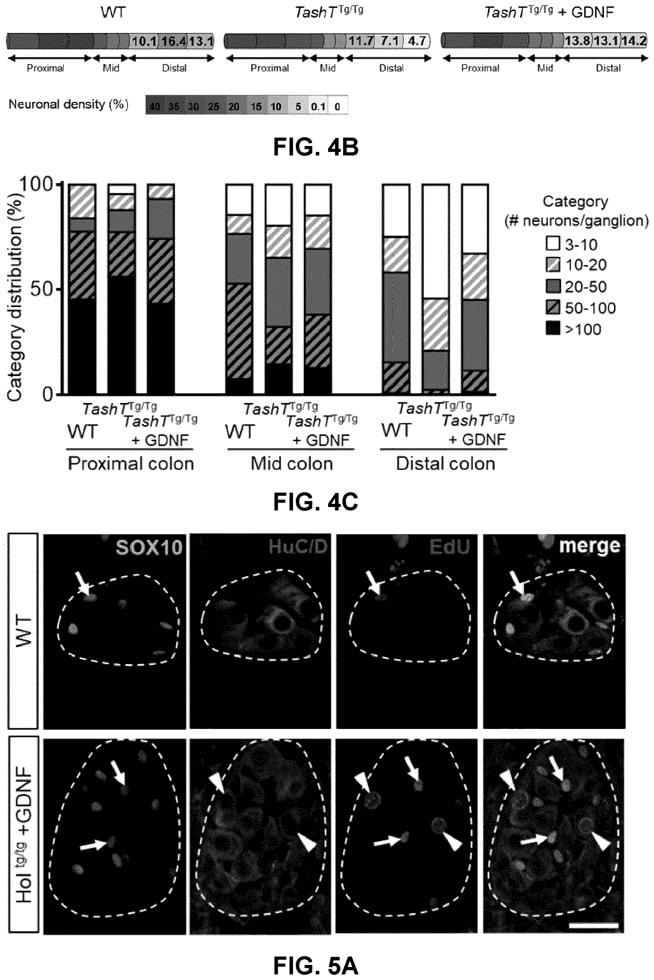

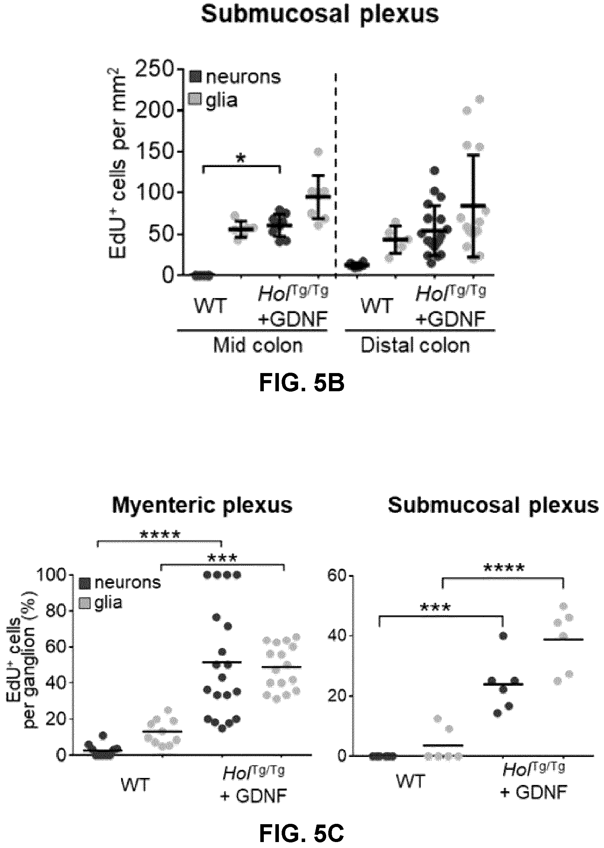

**FIGs. 4A-C**show an analysis of myenteric ganglion size and neuronal density in the colon of P20 *Hol*^{*Tg*/*Tg*} and *TashT*^{*Tg*/*Tg*} mice that were treated or not with GDNF between P4-P8. **(****FIG. 4A****)** Analysis of myenteric ganglion size *in Hol*^{*Tg*/*Tg*} mice. **(****FIG. 4B****)** Analysis of neuronal density in *TashT*^{*Tg*/*Tg*} mice. The average neuronal density is indicated for each colon sub-region (represented by cylinders) along the length of the colon. Neuronal density is expressed as the percentage of area occupied by HuC/D⁺ cells in a single focal plane at the level of the myenteric plexus within the bowel wall (n=6 mice per group; 3 fields of view per sub-region). For each distal colon subregion, the neuronal density is also given as a numerical value. **(****FIG. 5C****)** Analysis of myenteric ganglion size *in TashT*^{*Tg*/*Tg*} mice.
**FIGs. 5A-C**show an analysis of EdU incorporation in myenteric and submucosal ganglia of the colon from P20 WT and GDNF-treated *Hol*^{*Tg*/*Tg*} mice that were administered EdU between P4-P8. **(****FIG. 5A****)** Example of EdU incorporation in a z-stack projection of submucosal neurons (arrowheads) and glia (arrows) in the distal colon. Dashed outline marks area occupied by a single ganglion. Scale bar, 50 µm. **(****FIG. 5B****)** Quantitative analysis of EdU incorporation in submucosal neurons (HuC/D+) and glia (SOX10⁺) in mid and distal colon. Results are expressed as the number of EdU⁺ cells per mm² (n=3 WT and 3 GDNF-treated *Hol*^{*Tg*/*Tg*} mice; 2-7 fields of view per animal; **P*<0.05; one-way ANOVA with post-hoc Sidak's test). **(****FIG. 5C****)** Quantitative analysis of EdU incorporation in myenteric (left panel) and submucosal (right panel) neurons (HuC/D⁺) and glia (SOX10⁺) in distal colon. Results are expressed in percentage of EdU⁺ cells per ganglion (n=3 WT and 3 GDNF-treated *Hol*^{*Tg*/*Tg*} mice; 2-7 fields of view per animal; ****P*<0.001; *****P*<0.0001; one-way ANOVA with post-hoc Sidak's test). GDNF-treated mice received 10 µg GDNF in 10 µL enemas once daily from P4-P8.

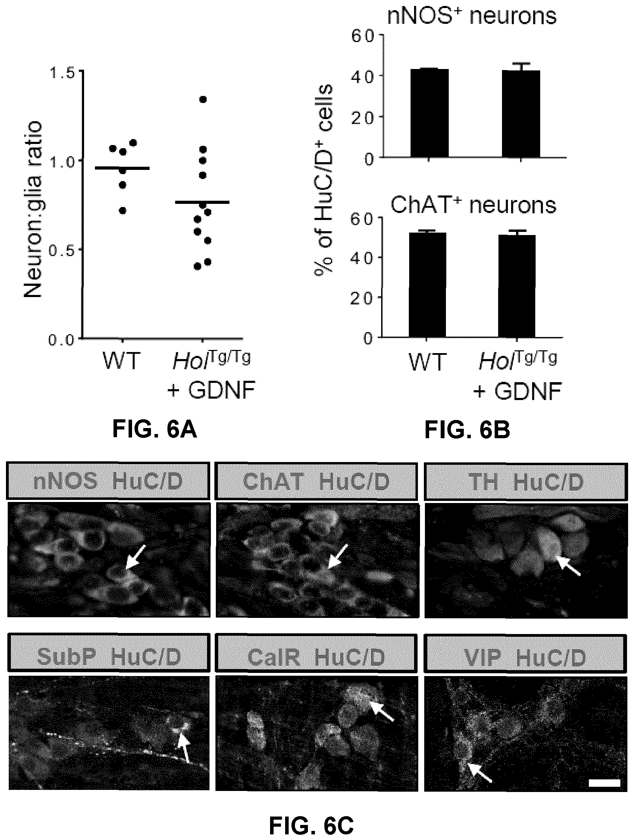

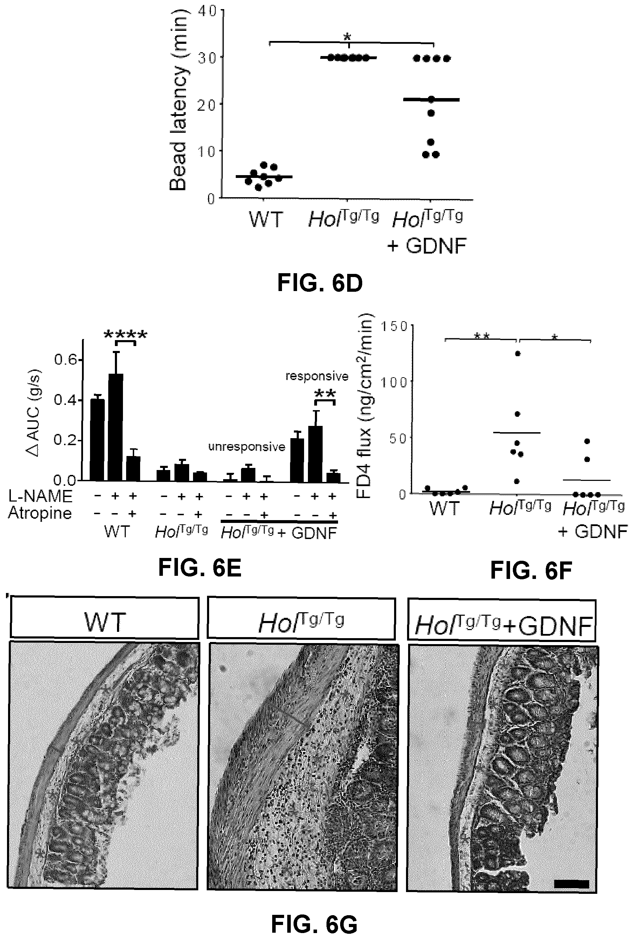

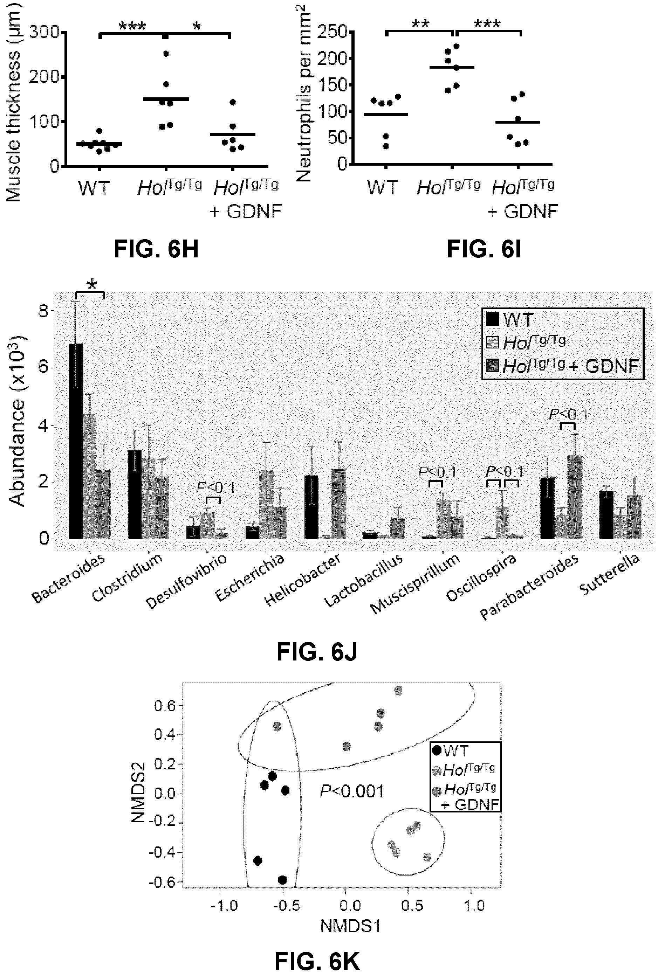

**FIGs. 6A-K** show the phenotypic and functional characterization of the GDNF-induced ENS in P20 *Hol*^{*Tg*/*Tg*} mice. **(****FIGs. 6A-B**Immunofluorescence-based quantitative analysis of GDNF-induced myenteric ganglia from the distal colon of *Hol*^{*Tg*/*Tg*} mice shows WT-like neuron (HuC/D⁺) to glia (Sox10⁺) ratio **(****FIG. 6A****), and** proportions of nitrergic (nNOS⁺ HuC/D⁺) and cholinergic (ChAT⁺ HuC/D⁺) neurons **(****FIG. 6B****)** (n=6 mice per group; 3 fields of view per animal). **(****FIG. 6C****)** Analysis of GDNF-induced myenteric ganglia not only shows the presence of nitrergic and cholinergic neurons but also other neuron subtypes expressing either tyrosine hydroxylase (TH), substance P (SubP), calretinin (CalR), or vasoactive intestinal peptide (VIP). All images are single focal planes representative of observations made from 3 mice per subtype marker, with arrows pointing to examples of indicated neuron subtypes. Scale bar, 20 µm. **(****FIG. 6D****)** *In vivo* bead latency test indicates partial recovery of colonic motility in some GDNF-treated *Hol*^{*Tg*/*Tg*} mice (n=8-9 mice per group, **P*<0.05; one-way ANOVA with post-hoc Sidak's test). Note that time to expel the glass bead after rectal insertion was capped at 30 min. in order to simplify the analysis without impacting statistical significance. **(****FIG. 6E****)** *Ex vivo* analysis of electric field-stimulated and drug-modulated patterns of longitudinal smooth muscle contraction-relaxation in an organ bath equipped with a force transducer. Contractile strength (expressed in g/s) is calculated from the difference from baseline of the area under the curve (AUC) values obtained after electric field stimulation. Mean ΔAUC values for the *Hol*^{*Tg*/}*^{Tg} +* GDNF group are divided into two sub-groups as a function of contractility response. About half of the colonic muscle strips from GDNF-treated *Hol*^{*Tg*/*Tg*} mice (4 out of 7) showed a WT-like pattern of response to sequential inhibition of nitrergic and cholinergic signaling (using L-NAME and atropine, respectively), whereas the other half (3 out of 7) remained poorly responsive similar to muscle strips from untreated *Hol*^{*Tg*/*Tg*} mice (n=6 WT and *Hol*^{*Tg*/}*^{Tg},* n=7 *Hol*^{*Tg*/*Tg*} + GDNF; ***P*<0.01; *****P*<0.0001; two-way ANOVA with post-hoc Tukey's test). **(****FIG. 6F****)** *Ex vivo* measurement of distal colon mucosal permeability to FITC-labeled 4kDa dextran (FD4) in Ussing chambers indicate increased permeability in *Hol*^{*Tg*/*Tg*} colons that is reversed in two-thirds (4 out of 6) of GDNF-treated *Hol*^{*Tg*/*Tg*} mice (n=6 mice per group; *P<0.05; ***P*<0.01; one-way ANOVA with post-hoc Sidak's test). **(****FIG. 6G-I**H&E staining of transverse sections of the distal colon shows that the GDNF treatment rescues both the increased thickness of smooth muscles (see brackets in **FIG. 6G** and quantification in **FIG. 6H****)** and the increased number of neutrophils in the submucosa (see asterisks in **FIG. 6G** and quantification in **FIG. 6I****)** (n=6 mice per group; Scale bar, 150µm; **P*<0.05; ***P*<0.01*;* ****P*<0.001; one-way ANOVA with post-hoc Sidak's test). **(****FIGs. 6J-K**Microbiome profiling shows that GDNF treatment at least partially rescues the microbiota imbalance normally observed in untreated *Hol*^{*Tg*/*Tg*} mice (n=5 mice per group). The bar histograms **(****FIGs. 6J****)** display the average relative abundance of 16S rRNA gene sequences at the genera level (**P*<0.05; one-way ANOVA with post-hoc Tukey's test). Beta-diversity comparisons **(****FIG. 6K****)** with 95% confidence interval ellipses are based on non-metric multidimensional scaling (NMDS) of Bray-Curtis dissimilarity of the relative abundance of operational taxonomic units among samples (*P*<0.001; PERMANOVA).

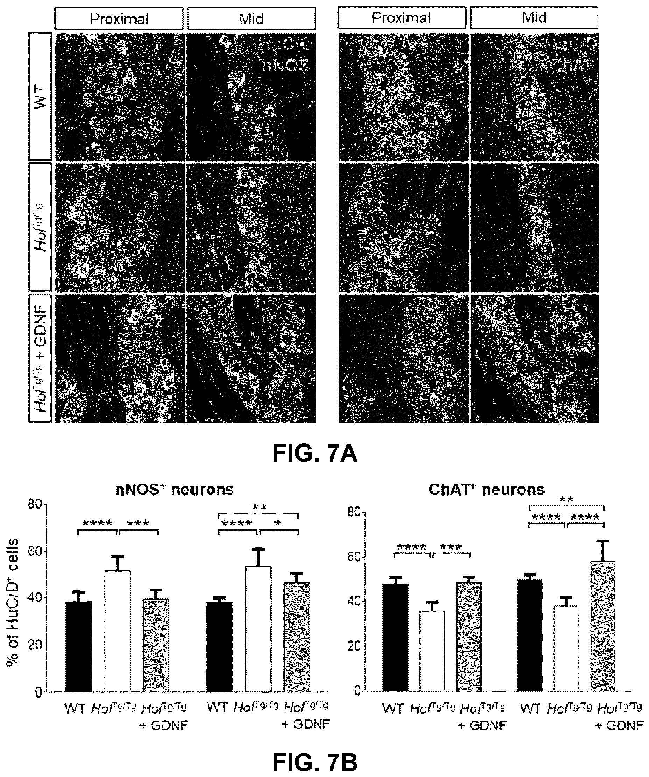

**FIGs. 7A-B**show the proportion of nitrergic and cholinergic myenteric neurons in the proximal and mid colon of WT, untreated *Hol*^{*Tg*/*Tg*} or GDNF-treated *Hol*^{*Tg*/*Tg*} mice at P20. **(****FIG. 7A****)** Qualitative analysis of the proportion of nitrergic (left panel) and cholinergic (right panel) neurons. Scale bar, 50 µm. **(****FIG. 7B****)** Quantitative analysis of the proportion of nitrergic (left panel) and cholinergic (right panel) neurons (n=3 WT and 3 GDNF-treated *Hol*^{*Tg*/*Tg*} mice; 3 fields of view per animal; **P*<0.05; ***P<*0.01; ****P*<0.001; *****P*<0.0001; one-way ANOVA with post-hoc Sidak's test). GDNF-treated mice received 10 µg GDNF in 10 µL enemas once daily from P4-P8.

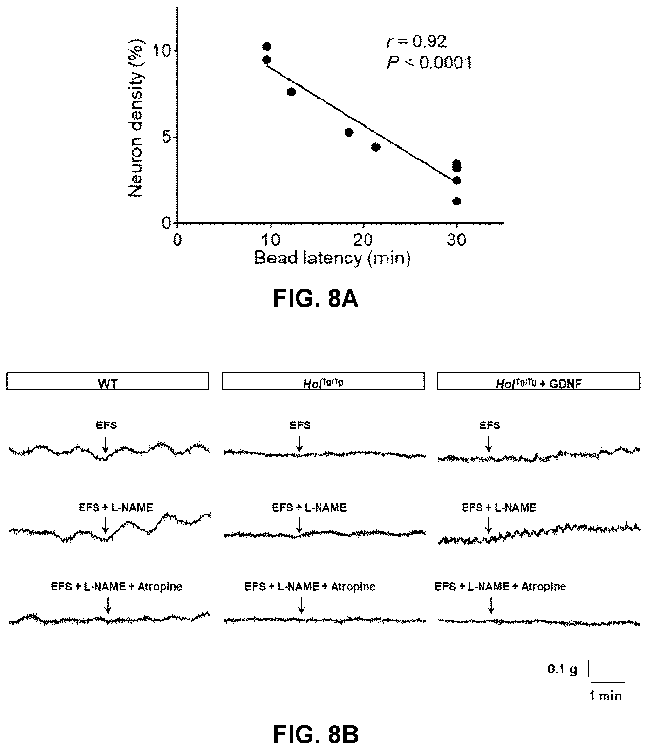

**FIGs. 8A-B**show supporting information for *in vivo* and *ex vivo* analyses of motility in the distal colon of WT, untreated *Hol*^{*Tg*/*Tg*} or GDNF-treated *Hol*^{*Tg*/*Tg*} mice at P20. **(****FIG. 8A****)** Correlation between neuron density in distal colon and time for bead expulsion in GDNF-treated *Hol*^{*Tg*/*Tg*} mice at P20 (in support of **FIG. 6D****). (****FIG. 8B****)** Examples of electric field-stimulated and drug-modulated patterns of longitudinal smooth muscle contraction-relaxation in an organ bath equipped with a force transducer (in support of **FIG. 6E****).** In responsive tissues, electric field stimulation (EFS) triggers contractions of colonic muscles that can be slightly increased by L-NAME-mediated inhibition of nitrergic signaling, and robustly counteracted by atropine-mediated inhibition of cholinergic signaling. GDNF-treated mice received 10µg GDNF in 10µL enemas once daily from P4-P8.

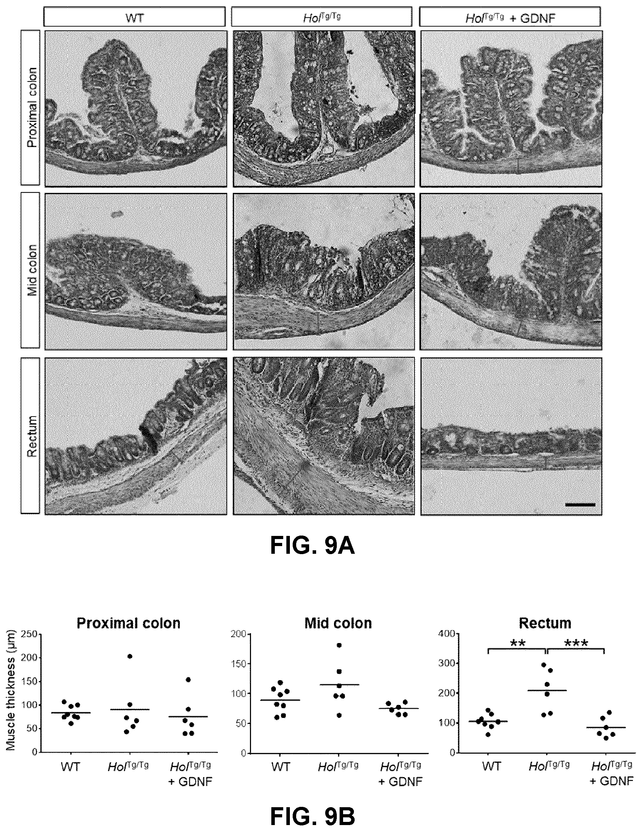

**FIGs. 9A-B**show an analysis of smooth muscle thickness in the distal colon of WT, untreated *Hol*^{*Tg*/*Tg*} or GDNF-treated *Hol*^{*Tg*/*Tg*} mice at P20. **(****FIG. 9A****)** Representative H&E-stained cross-sections of different colon segments, with smooth muscle thickness indicated by red brackets. Scale bar, 150 µm. **(****FIG. 9B****)** Average muscle thickness for each colon segment (n=6 mice per group; ***P*<0.01; ****P*<0.001; one-way ANOVA with post-hoc Tukey's test). GDNF-treated mice received 10 µg GDNF in 10 µL enemas once daily from P4-P8.

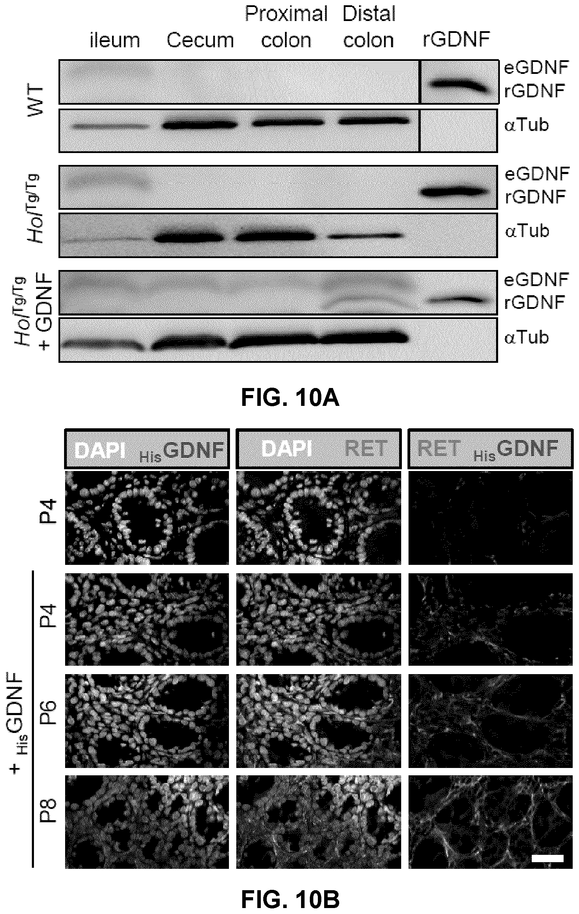

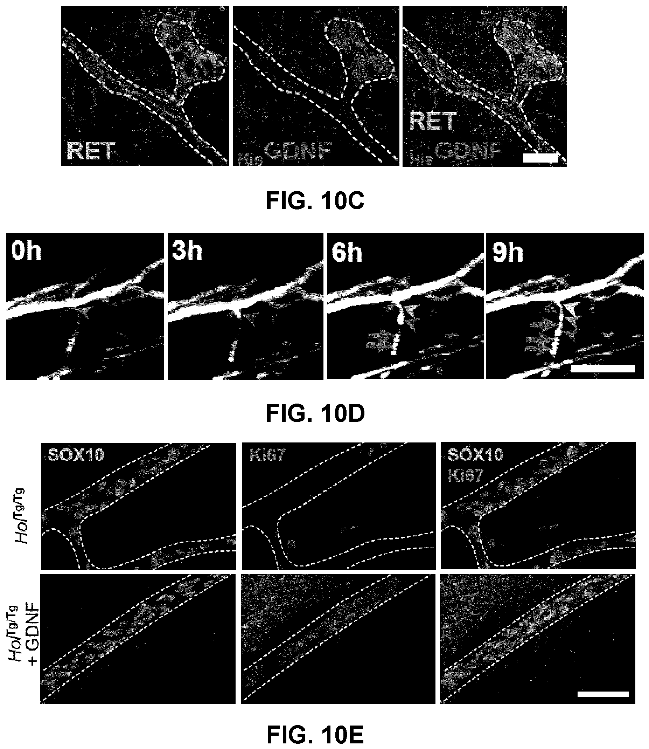

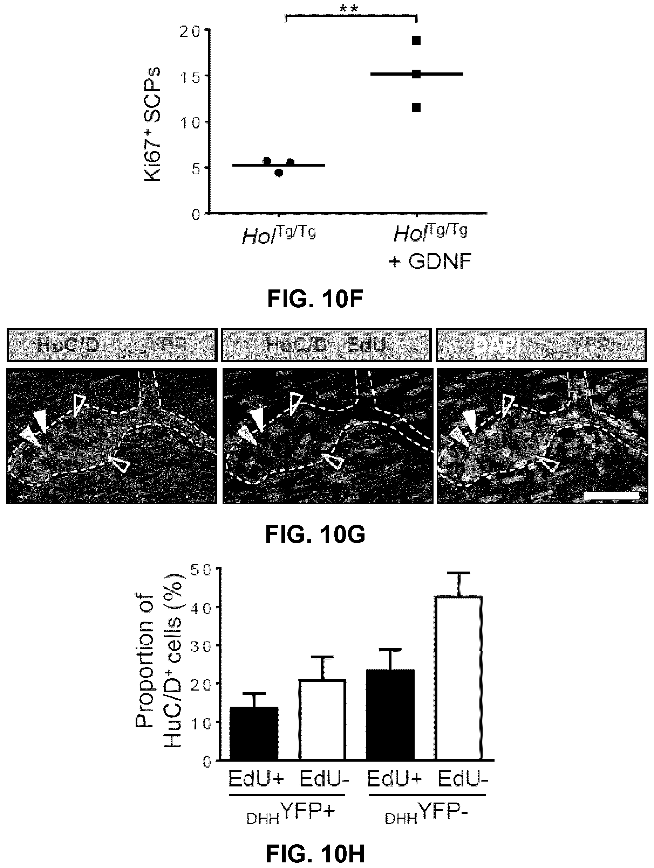

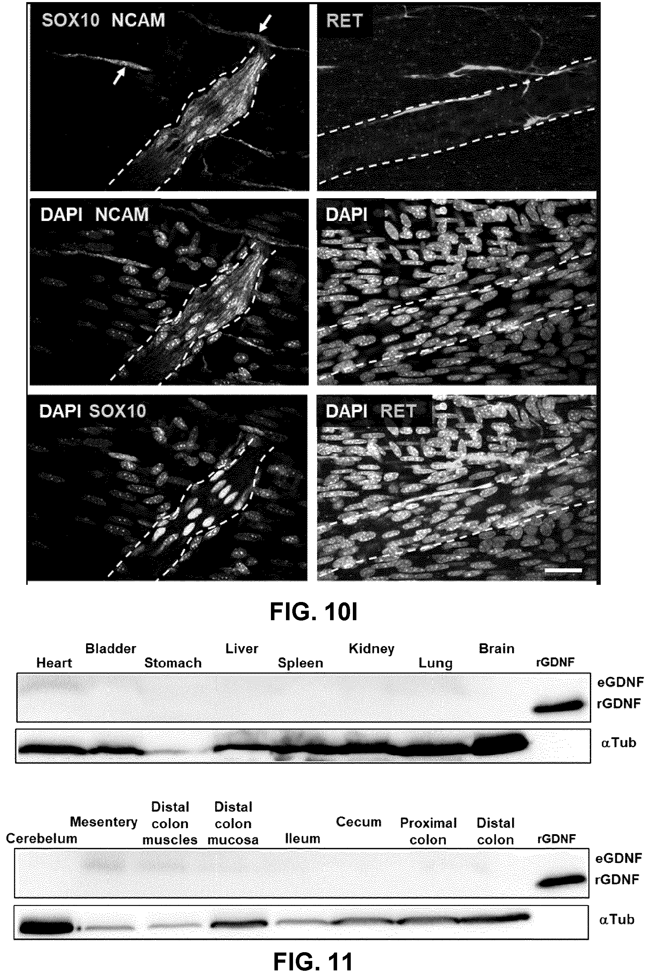

**FIGs. 10A-I** show that extrinsic Schwann cell precursors (SCPs) are a source of GDNF-induced neurons and glia in the otherwise aganglionic colon. **(****FIG. 10A****)** Western blot analysis of GDNF distribution in different sub-regions of the GI tract from WT, *Hol*^{*Tg*/*Tg*} and GDNF-treated *Hol*^{*Tg*/*Tg*} mice at P8. Endogenous GDNF (eGDNF) is normally restricted to the ileum in all mice whereas recombinant GDNF (rGDNF) is exclusively detected in the distal colon of GDNF-treated *Hol*^{*Tg*/*Tg*} mice. In GDNF-treated *Hol*^{*Tg*/}*^{Tg},* eGDNF is expressed in all sub-regions of the colon. The displayed anti-GDNF blots are representative of observations made from 3 mice per group. (FIGs. **10B-C**Time-course analysis of the distribution of a 6xHis-tagged version of GDNF (_{His}GDNF) used for enema treatments of *Hol*^{*Tg*/*Tg*} mice between P4-P8. Anti-His and anti-RET double staining of the distal colon at 2-day intervals shows that both _{His}GDNF and RET accumulate in the submucosa during the treatment **(****FIG. 10B****).** Both are also detected in induced myenteric neurons close to extrinsic nerve fibers at P8 **(****FIG.** 10C). All images show a z-stack projection representative of observations made from 3 mice. Scale bar, 20 µm. **(****FIG. 10D****)** Representative images from 10-hour long time-lapse recordings of aganglionic colon tissues from *Hol*^{*Tg*/*Tg*}; *G4-RFP* mice showing that SCP-like cells are dividing (arrows) and migrating (arrowheads) on extrinsic nerve fibers. Explants were prepared from P4 distal colons and pre-cultured for 72h with GDNF before live imaging on a confocal microscope in the continued presence of GDNF. Images are projections of 50 µm-thick z-stacks representative of observations made from 3 explants. Scale bar, 100µm. **(****FIGs. 10E-F**Anti-SOX10 and anti-Ki67 double labeling demonstrates that exposure to GDNF for 96h markedly increases the rate of SCP proliferation in explants of distal colon prepared from P4 *Hol*^{*Tg*/*Tg*} mice. Images in **FIG. 10E** are single focal planes representative of observations made from 3 explants. Scale bar, 50 µm. Each value in **FIG. 10F** corresponds to the average percentage of Ki67⁺ SCPs (i.e., (Ki67⁺SOX10⁺ / SOX10⁺) x 100) calculated from a minimum of 3 fields of view per explant (***P*<0.01; two-tailed Student's *t*-test). **(****FIGs. 10G-H** Immunofluorescence analysis of myenteric ganglia in the distal colon of P20 *Hol*^{*Tg*/*Tg*}; *Dhh-Cre*^{Tg/}*⁺;R26*^{YFP/*+*} mice that were administered GDNF enemas and EdU via intraperitoneal injections between P4-P8. Four categories of induced neuron are detected: 1) SCP-derived (*Dhh*+ lineage) and EdU-positive (filled grey arrowhead); 2) SCP-derived and EdU-negative (empty grey arrowhead); 3) unknown origin and EdU-positive (filled white arrowhead); 4) unknown origin and EdU-negative (empty white arrowhead). Images in **FIG. 10G** are single focal planes representative of observations made from 3 mice. Scale bar, 50 µm. The relative proportions of the four categories of induced neurons per ganglion plotted in **FIG. 10H** corresponds to the averages calculated from a minimum of 3 fields of view per mouse. Dashed outlines mark area occupied by either an extrinsic nerve fiber **(****FIG. 10E****),** or an extrinsic nerve fiber and an adjacent single ganglion **(****FIGs. 10C** and **G).** **FIG. 10I****:** Schwann cells in the aganglionic distal colon of *Hol*^{Tg/Tg} mice express neural cell adhesion molecule (NCAM) but not RET. Immunofluorescence analysis of NCAM and RET expression in extrinsic nerve fibers (delineated by *dashed lines*) from the distal colon of untreated *Hol*^{Tg/Tg} mice at P20. NCAM but not RET is expressed in SOX10⁺ Schwann cells and putative enteric glia/ENS progenitors (*arrows*)*.* DAPI, 4',6-diamidino-2-phenylindole. The displayed images are single focal planes representative of observations made from 3 mice. Scale bar, 50 µm.
**FIG. 11** shows an analysis of GDNF distribution in multiple tissues of GDNF-treated *Hol*^{*Tg*/*Tg*} mice at P20. Western bolt analysis of αTubulin-normalized levels of endogenous GDNF (eGDNF) and recombinant GDNF (rGDNF) in different tissues of P20 *Hol*^{*Tg*/*Tg*} mice that received 10 µg GDNF in 10 µL enemas once daily from P4-P8. The displayed blots are representative of observations made from 3 mice.

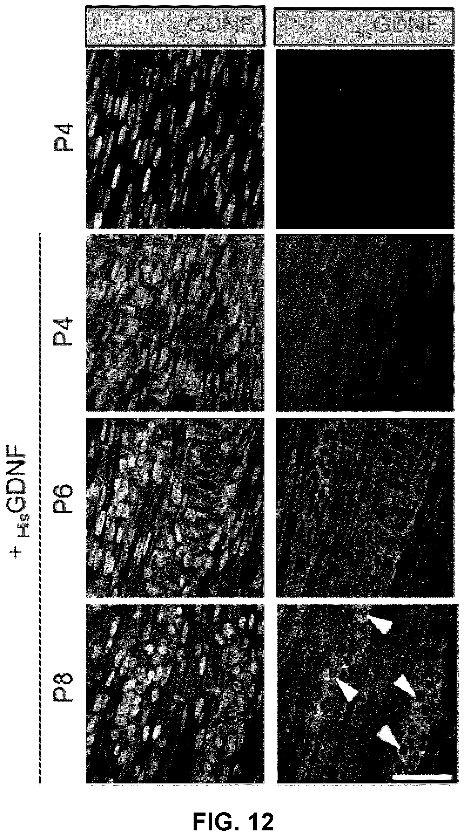

**FIG. 12** shows a time-course analysis of _{His}GDNF distribution and RET expression in colonic smooth muscles of P4-P8 *Hol*^{*Tg*/*Tg*} mice treated with _{His}GDNF. Immunofluorescence analysis of _{His}GDNF distribution and RET expression in distal colon muscularis of _{His}GDNF-treated *Hol*^{*Tg*/*Tg*} mice. White arrowheads point to RET⁺ neurons that also stain positive for _{His}GDNF. All images show a single focal plane representative of observations made from 3 mice. Scale bar, 20 µm.

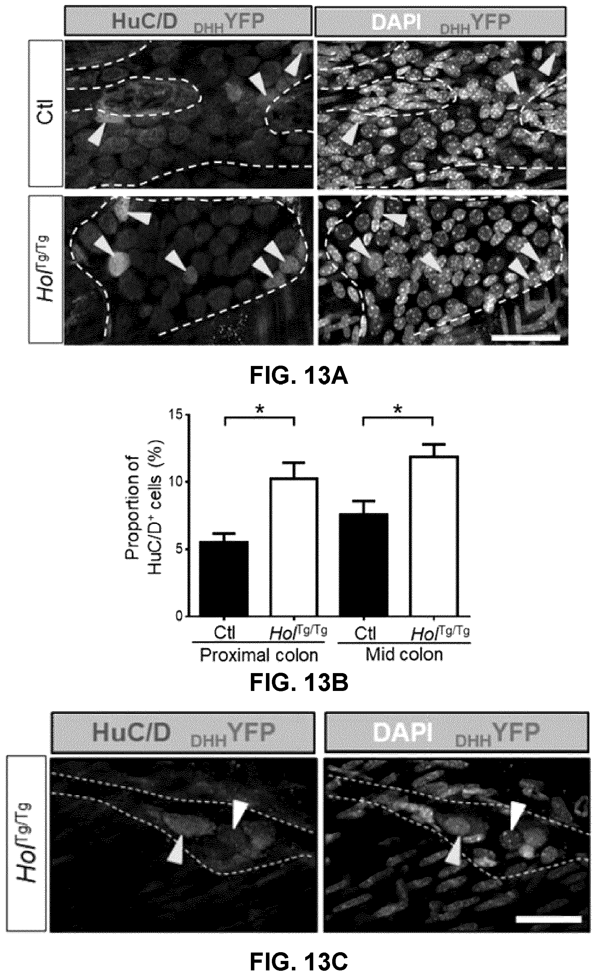

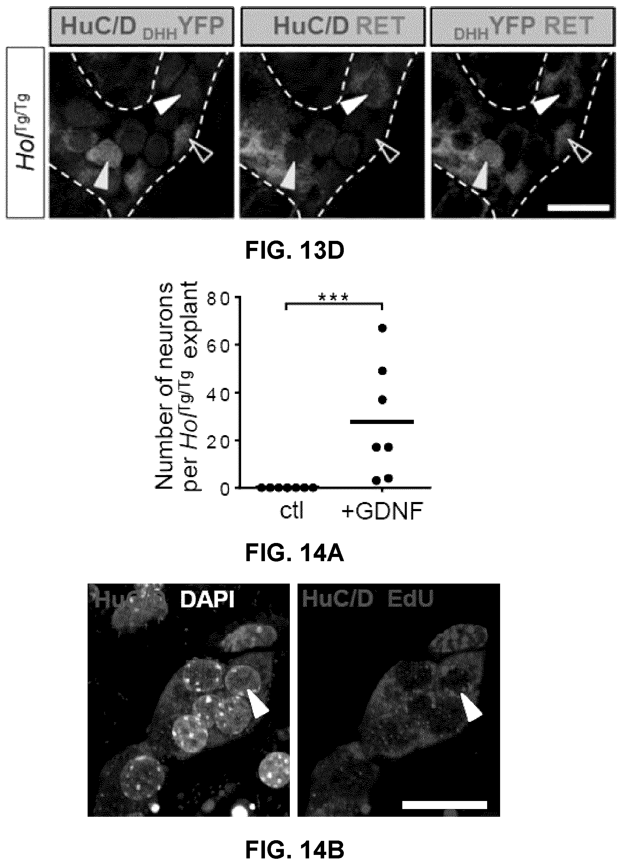

**FIGs. 13A-D**show an analysis of SCP-derived neurogenesis in myenteric and submucosal ganglia of *Dhh-Cre*^{*Tg*l+}*;R26*^{YFP/+} and *Hol*^{*Tg*/*Tg*};*Dhh-Cre*^{Tg/+};*R26*^{YFP/+} mice at P20. **(****FIG. 13A****)** Analysis of myenteric neurons (HuC/D⁺) and YFP expression in the proximal colon of *Dhh-Cre*^{*T*g/+};*R26*^{*Y*FP/+} (Ctl) and *Hol*^{*Tg*/*Tg*};*Dhh-Cre*^{Tg/+};*R26*^{YFP/+} (*Hol*^{*Tg*/*Tg*}) mice. Yellow arrowheads point to SCP-derived neurons. **(****FIG. 13B****)** Quantitative analyses of myenteric neurons (HuC/D⁺) and YFP expression in the proximal and mid-colon of *Dhh-Cre*^{Tg/+}; *R26*^{YFP/+} (Ctl) and *Hol*^{*Tg*/*Tg*};*Dhh-Cre*^{Tg/+};*R26*^{YFP/+} (*Hol*^{*Tg*/*Tg*}) mice (n=3 Ctl and 3 *Hol*^{*Tg*/*Tg*} mice; 3 fields of view per animal; **P*<0.05; one-way ANOVA with post-hoc Sidak's test). **(****FIG. 13C****)** Analysis of submucosal neurons (HuC/D⁺) and YFP expression in the distal colon of *Hol*^{*Tg*/*Tg*};*Dhh-Cre*^{Tg/+};*R26*^{YFP/+} (*Hol*^{*Tg*/*Tg*}) mice that were treated with GDNF between P4-P8. Neurons of either SCP (grey arrowhead) or unknown (white arrowhead) origin are detected. **(****FIG. 13D****)** Analysis of RET-expressing myenteric neurons (HuC/D⁺) and YFP expression in the distal colon of *Hol*^{*Tg*/*Tg*};*Dhh-Cre*^{Tg/+};*R26*^{YFP/+} (*Hol*^{*Tg*/*Tg*}) mice that were treated with GDNF between P4-P8. RET is expressed in a subset of neurons, regardless of SCP (RET+, filled grey arrowhead; RET-, empty grey arrowhead) or non-SCP (white arrowhead) origin. All displayed images are z-stack projections representative of observations made from 3 mice. Scale bar, 50 µm. Dashed outline marks area occupied by a single ganglion.

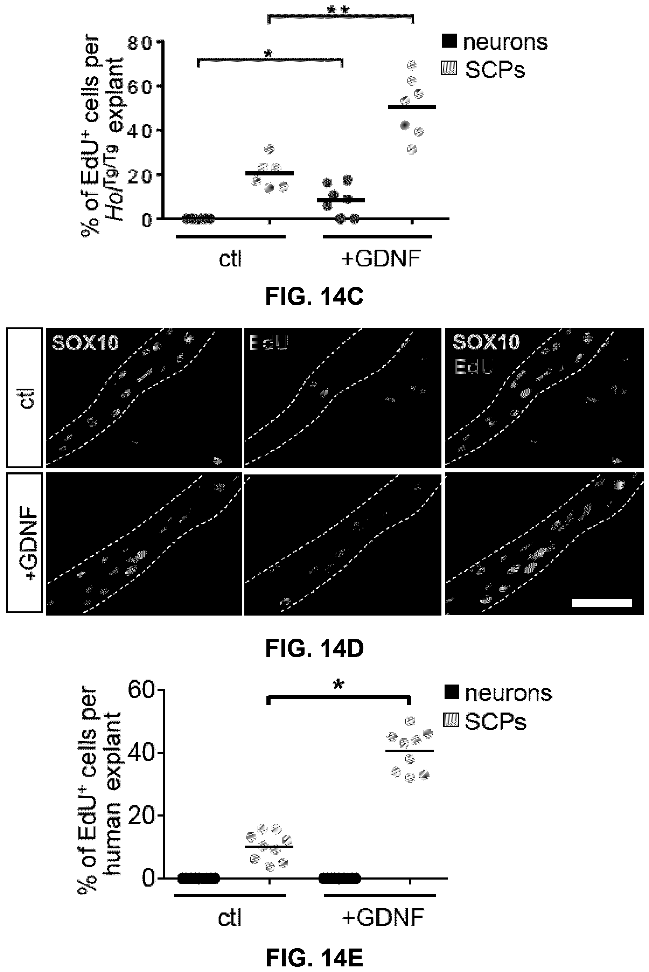

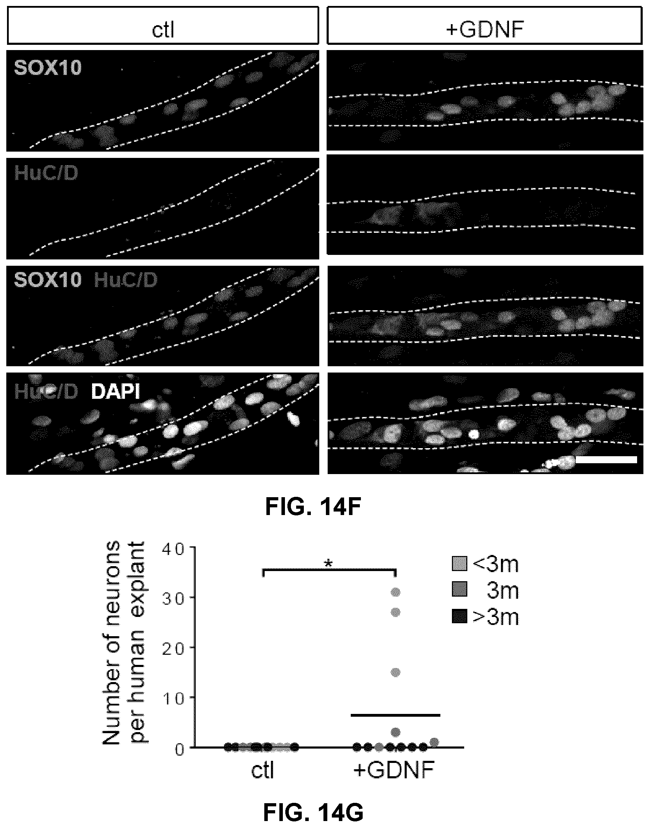

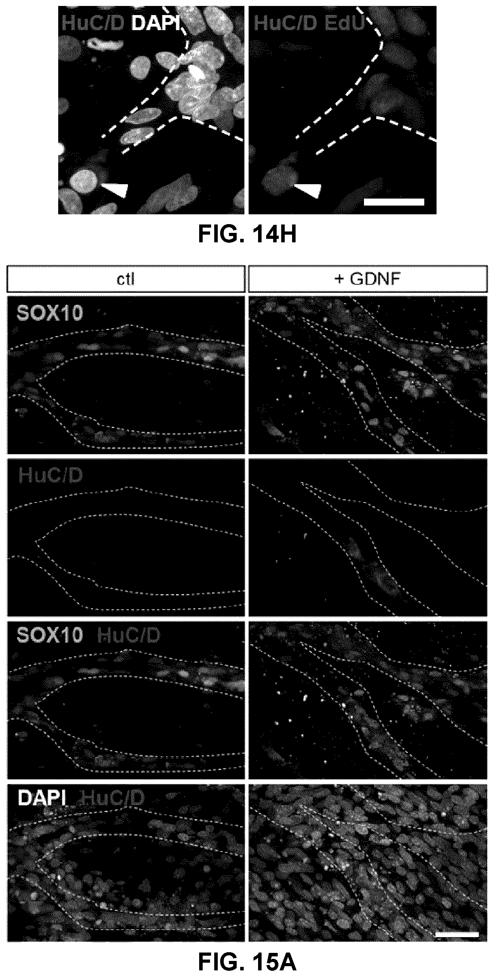

**FIGs. 14A-H**show the *ex vivo* preclinical testing of GDNF therapy on explants of aganglionic colon from *Hol*^{*Tg*/*Tg*} mice and human HSCR patients. **(****FIGs. 14A-C)** Immunofluorescence-based analysis of explants prepared from the distal colon of P4 *Hol*^{*Tg*/*Tg*} mice, and cultured for 96h in presence of GDNF and EdU (+GDNF) or EdU alone (ctl). New HuC/D⁺ neurons can be induced by GDNF under these *ex vivo* culture conditions but the total number of neurons per explant is variable **(****FIG. 14A****),** these neurons only formed very small ganglia, if any **(****FIG. 14B****),** and they were less likely to show EdU incorporation than SCPs (see arrowhead in **FIG. 14B** and quantification in **FIG. 14C****)** (n=7 explants per condition; *P<0.05; ***P<0.01; ***P<0.001;* two-tailed Mann-Whitney U test). **(****FIGs. 14D-G**Immunofluorescence-based analysis of explants prepared from samples of aganglionic colon resected from human HSCR patients, and cultured for 96h in presence of GDNF and EdU (+GDNF) or EdU alone (ctl). For all human explants cultured under these conditions GDNF treatment leads to robust incorporation of EdU in SOX10⁺ SCPs but not in HuC/D⁺ neurons **(****FIGs. 14D-E)****.** A significant number of HuC/D⁺ neurons can be induced by GDNF in a subset of explants **(****FIG. 14F****),** which all originated from patients less than 3 months of age at the time of surgery **(****FIG. 14G****)** (n=12 explants per condition; *P<0.05; two-tailed Mann-Whitney U test). **(****FIG. 14H****)** Extended culture in presence of GDNF for a total of 7 days allowed the detection of neurons in human explants from patients ≥3 months of age at the time of surgery, including some that incorporated EdU (arrowhead). All displayed images represent a z-stack projection at the level of the myenteric plexus. Scale bars, 50 µm **(****FIGs. 14B** and **H)** and 100 µm **(****FIGs. 14D** and F). Dashed outline marks area occupied by extrinsic nerve fibers.

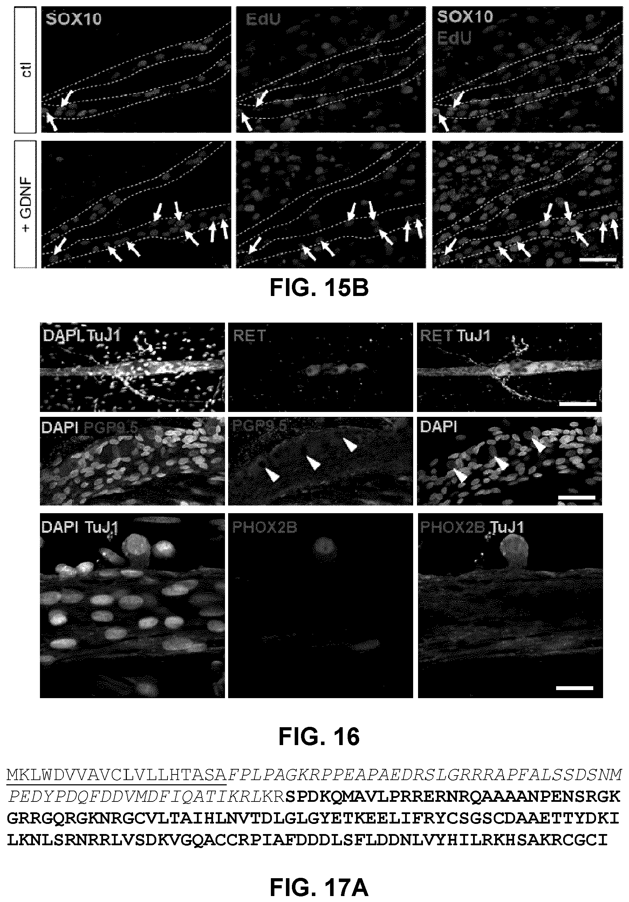

**FIGs. 15A-B**show an analysis of neurogenesis and SCP proliferation in distal colon explants prepared from P4 *Hol*^{*Tg*/*Tg*} mice and cultured in presence or absence of GDNF for 96h. Representative images of HuC/D⁺ neurons **(****FIG. 15A****),** and EdU⁺ SOX10⁺ proliferating SCPs (arrows in **FIG. 15B****)** in explants of distal colon from *Hol*^{*Tg*/*Tg*} mice cultured in presence of GDNF and EdU (+GDNF) or EdU alone (ctl). The displayed images are single focal planes representative of observations made from 7 mice. Scale bar, 50 µm. Dashed outline marks area occupied by extrinsic nerve fibers.
**FIG. 16** shows a marker analysis of GDNF-induced neurons in sigmoid colon explants prepared from HSCR patients and cultured in presence of GDNF for 96h. Immunofluorescence analysis showing that human GDNF-induced neurons are closely associated with extrinsic nerves and express βIII-Tubulin (TuJ1), RET, PGP9.5 and PHOX2B (in support of **FIG. 14F****).** Arrowheads point to round/ovoid nuclei of PGP9.5⁺ neurons. The displayed images are single focal planes representative of observations made from 3 human samples. Scale bar, 100 µm (upper panels), 50 µm (middle panels) and 25 µm (lower panels).

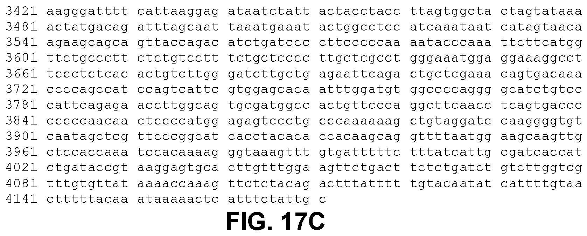

**FIG. 17A** shows the amino acid sequence of human GDNF isoform 1 (UniProtKB accession No. P39905, SEQ ID NO:1), with the sequence corresponding to the signal peptide underlined (residues 1-19), the sequence corresponding to the propeptide italicized (residues 20-75) and the sequence corresponding to the mature polypeptide in bold (residues 78-211).

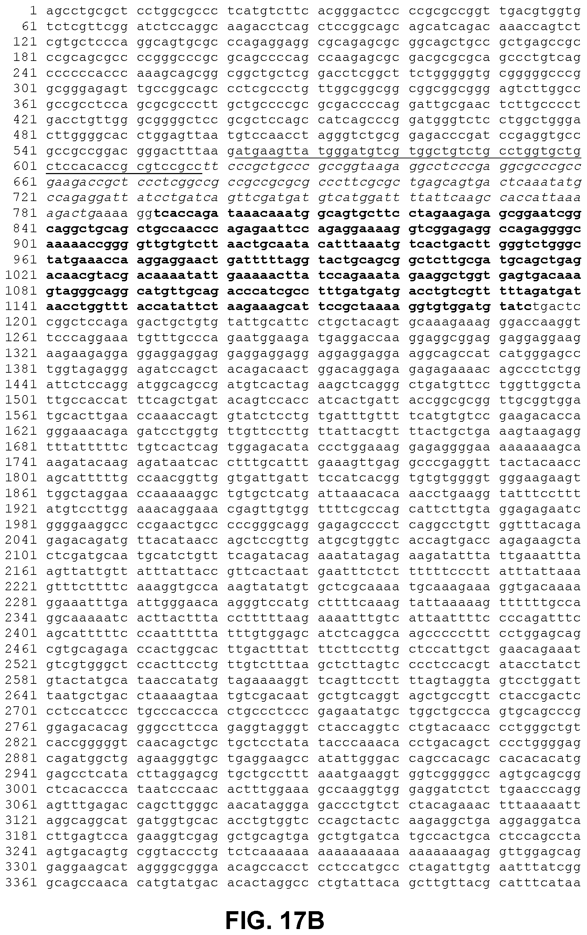

**FIGs. 17B-C** show the nucleotide sequence of the cDNA encoding human GDNF isoform 1 (RefSeq accession No. NM_000514.4, SEQ ID NO:2), with the sequence encoding the signal peptide underlined (nucleotides 562-618), the sequence encoding the propeptide italicized (nucleotides 619-786) and the sequence encoding the mature polypeptide in bold (nucleotides 793-1194).

### DISCLOSURE OF INVENTION

The use of the terms "a" and "an" and "the" and similar referents in the context of describing the technology (especially in the context of the following claims) are to be construed to cover both the singular and the plural, unless otherwise indicated herein or clearly contradicted by context.

The terms "comprising", "having", "including", and "containing" are to be construed as open-ended terms (i.e., meaning "including, but not limited to") unless otherwise noted.

All methods described herein can be performed in any suitable order unless otherwise indicated herein or otherwise clearly contradicted by context.

The use of any and all examples, or exemplary language ("*e*.*g*.", "such as") provided herein, is intended merely to better illustrate embodiments of the claimed technology and does not pose a limitation on the scope unless otherwise claimed.

No language in the specification should be construed as indicating any non-claimed element as essential to the practice of embodiments of the claimed technology.

Herein, the term "about" has its ordinary meaning. The term "about" is used to indicate that a value includes an inherent variation of error for the device or the method being employed to determine the value, or encompass values close to the recited values, for example within 10% of the recited values (or range of values).

Recitation of ranges of values herein are merely intended to serve as a shorthand method of referring individually to each separate value falling within the range, unless otherwise indicated herein, and each separate value is incorporated into the specification as if it were individually recited herein. All subsets of values within the ranges are also incorporated into the specification as if they were individually recited herein.

Where features or aspects of the disclosure are described in terms of Markush groups or list of alternatives, those skilled in the art will recognize that the disclosure is also thereby described in terms of any individual member, or subgroup of members, of the Markush group or list of alternatives.

Unless specifically defined otherwise, all technical and scientific terms used herein shall be taken to have the same meaning as commonly understood by one of ordinary skill in the art (e.g., in stem cell biology, cell culture, molecular genetics, immunology, immunohistochemistry, protein chemistry, and biochemistry).

Unless otherwise indicated, the recombinant protein, cell culture, and immunological techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T. A. Brown (editor), Essential Molecular Biology: A Practical Approach, Volumes 1 and 2, IRL Press (1991), D. M. Glover and B. D. Hames (editors), DNA Cloning: A Practical Approach, Volumes 1-4, IRL Press (1995 and 1996), and F. M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J. E. Coligan et al. (editors) Current Protocols in Immunology, John Wiley & Sons (including all updates until present).

In the studies described herein, the present inventors show that administration of a proper dosage of recombinant GDNF in the distal colon via rectal enema can induce permanent formation of new functioning enteric neurons and glia in otherwise aganglionic colon, and restoration of colon motility, in HSCR mouse models. Genetic lineage tracing show that SCPs located in extrinsic nerve fibers are one source for these newly generated enteric neurons and glia. It is further demonstrated that GDNF can stimulate neurogenesis in cultured explants of aganglionic colon from human HSCR patients. Thus, GDNF appeared as a primary candidate for postnatal reactivation of ENS progenitors in the aganglionic zone notably because of its ability to stimulate migration and proliferation of Schwann cells in a RET-independent but GFRα1-dependent manner through its alternative receptor neural cell adhesion molecule (NCAM). These results provide evidence that administration of recombinant GDNF administered in the colon (e.g., distal colon) may be used for the treatment for enteric neuropathies (e.g., ENS defects such as HSCR) i.e. for improving one or more of the pathological features of enteric neuropathies, in human patients.

Accordingly, the present description provides a method for inducing enteric neurogenesis in an aganglionic or hypoganglionic segment of the distal colon of a human subject suffering from an enteric neuropathy (*e.g.,* Hirschsprung disease (HSCR) or intestinal hypoganglionosis), the method comprising administrating a pharmaceutical composition comprising an effective dose of a Glial cell line-Derived Neurotrophic Factor (GDNF) polypeptide and a pharmaceutically acceptable carrier into the distal colon of the subject.

The present description also provides a method for restoring distal colon motility and/or epithelial barrier in a human subject suffering from an enteric neuropathy (*e.g.,* HSCR or intestinal hypoganglionosis), the method comprising administrating a pharmaceutical composition comprising an effective dose of a GDNF polypeptide and a pharmaceutically acceptable carrier into the distal colon of the subject.

The present description also provides the use of a pharmaceutical composition comprising a human GDNF polypeptide and a pharmaceutically acceptable carrier for inducing enteric neurogenesis in an aganglionic or hypoganglionic segment of the distal colon of a human subject suffering from an enteric neuropathy (*e.g.,* HSCR or intestinal hypoganglionosis), wherein the composition is for administration into the distal colon of the subject.

The present description also provides the use of a pharmaceutical composition comprising a human GDNF polypeptide and a pharmaceutically acceptable carrier for restoring distal colon motility in a human subject suffering from an enteric neuropathy (*e.g.,* HSCR or intestinal hypoganglionosis), wherein the composition is for administration into the distal colon of the subject.

The present description also provides the use of a pharmaceutical composition comprising a human GDNF polypeptide and a pharmaceutically acceptable carrier for the manufacture of a medicament for inducing enteric neurogenesis in an aganglionic or hypoganglionic segment of the distal colon of a human subject suffering from an enteric neuropathy (*e.g.,* HSCR or intestinal hypoganglionosis), wherein the medicament is for administration into the distal colon of the subject.

The present description also provides the use of a pharmaceutical composition comprising a human GDNF polypeptide and a pharmaceutically acceptable carrier for the manufacture of a medicament for restoring distal colon motility in a human subject suffering from an enteric neuropathy (*e.g.,* HSCR or intestinal hypoganglionosis), wherein the medicament is for administration into the distal colon of the subject.

The present description also provides a pharmaceutical composition for inducing enteric neurogenesis in an aganglionic or hypoganglionic segment of the distal colon of a human subject suffering from an enteric neuropathy (*e.g.,* HSCR or intestinal hypoganglionosis), the composition comprising a human GDNF polypeptide and a pharmaceutically acceptable carrier, and wherein the pharmaceutical composition is for administration into the distal colon of the subject.

The present description also provides a pharmaceutical composition for restoring distal colon motility in a human subject suffering from an enteric neuropathy (*e.g.,* HSCR or intestinal hypoganglionosis), the composition comprising a human GDNF polypeptide and a pharmaceutically acceptable carrier, and wherein the pharmaceutical composition is for administration into the distal colon of the subject.

The term "distal colon" as used herein refers to the last three portions of the colon, namely the descending colon, the sigmoid colon and the rectum. In an embodiment, the pharmaceutical composition is administered or is for administration into the rectum and/or the sigmoid colon. In an embodiment, the pharmaceutical composition is administered or is for administration into the rectosigmoid region, which comprises the last part of the sigmoid colon and the beginning of the rectum. The skilled person would understand that the pharmaceutical composition may be administered directly into the distal colon, or may be administered at a site away from the distal colon but using suitable means to provide delivery of the pharmaceutical composition (and more specifically of the human GDNF polypeptide) into the distal colon. For example, the pharmaceutical composition may comprise a coating that is specifically degraded under the conditions (*e.g.,* pH, enzymatic environment, bacterial environment, etc.) of the distal colon, and thus the pharmaceutical composition described herein may be administered in another region of the gastro-intestinal system but the human GDNF polypeptide will only be released once the pharmaceutical composition reaches the colon, and more specifically the distal colon. Approaches for colon specific drug delivery are well known in the art (see, *e.g.,* Philip et al., Oman Med J. 2010 Apr; 25(2): 79-87; Lee et al., Pharmaceutics. 2020 Jan; 12(1): 68), and include pH-dependent systems (*e.g.,* using pH-dependent polymers), receptor-mediated systems, magnetically-driven systems, delayed or time-dependent systems, microbially triggered drug delivery systems (*e.g.,* comprising sugar-based polymers that may be degraded by enzymes produced by the colon microflora such as glucoronidase, xylosidase, arabinosidase, galactosidase), pressure controlled colonic delivery capsule (drug release induced by the higher pressures encountered in the colon), osmotic controlled drug delivery, as well as any combinations of these approaches (*e.g.,* colon targeted delivery system (CODESTM) using a combined approach of pH dependent and microbially triggered drug delivery).

The term "enteric neuropathy" as used herein refers to a disease associated with abnormalities in the ENS, including abnormal development of the ENS, *e.g.,* abnormal number of neurons (hypoganglionosis, aganglionosis) and/or abnormal differentiation of neurons. Examples of enteric neuropathies include enteric dysganglionoses such as HSCR and intestinal hypoganglionosis. In particular, the enteric neuropathy may be HSCR. Alternatively, the enteric neuropathy may be intestinal hypoganglionosis.

The expression "inducing enteric neurogenesis" as used herein refers to an increase in the production of enteric neurons and/or enteric glial cells relative to prior to treatment with the composition comprising a human GDNF polypeptide. The enteric nervous system comprises various types of neurones including enteric primary afferent neurons (EPANs), excitatory circular muscle motorneurons, inhibitory circular muscle motorneurons, longitudinal muscle motorneurons, ascending interneurons, descending interneurons, secretomotor and vasomotor neurons, and intestinofugal neurons, as well as enteric glial cells (EGCs) that provide structural support to neurons and contribute to neuronal maintenance, survival, and function (Costa et al., Gut 2000;(Suppl IV) 47: iv15-iv19; De Giorgio et al., American Journal of Physiology-Gastrointestinal and Liver Physiology, Vol. 303, No. 8: G887-G893, 2012).

The production of one or more of these cell types may be induced by the administration/use of the composition comprising a human GDNF polypeptide. For example, the production of EGCs, preferably Sox10-expressing EGCs, is induced by the administration/use of the composition comprising a human GDNF polypeptide. For example, the administration/use of the composition comprising a human GDNF polypeptide restores the enteric neurons/glial cell ratio in the colon (*e.g.,* distal colon) of the patient. The enteric neurons/glial cell ratio in the colon (*e.g.,* distal colon) of the patient may be at least 0.5, *e.g.,* between 0.5 and 1.5. For example, the administration/use of the composition comprising a human GDNF polypeptide restores the proportions of nitrergic (nNOS⁺) and cholinergic (ChAT⁺) neurons in the colon (*e.g.,* distal colon) of the patient.

For example, the administration/use of the composition comprising a human GDNF polypeptide reduces the infiltration of inflammatory or immune cells (*e.g.,* neutrophils) in the colon (*e.g.,* distal colon). For example, the administration/use of the composition comprising a human GDNF polypeptide restores (partly or completely) the proportions of immune cells in the colon (*e.g.,* distal colon).

The term "human GDNF polypeptide" as used herein refers to the native mature human GDNF protein, or to functional variants or fragments thereof that retain a biological activity of the native mature human GDNF protein, *e.g.,* the ability to bind to a GDNF receptor (particularly the "rearranged during transfection" (RET) proto-oncogene and/or the Neural Cell Adhesion Molecule (NCAM) receptor) and trigger a signal in a cell expressing a GDNF receptor (*e.g.,* **RET** and/or NCAM). The amino acid sequence of native human GDNF protein (isoform 1, the canonical sequence) is depicted in **FIG. 17A** (SEQ ID NO:1), with the sequence corresponding to the mature protein (residues 78-211) highlighted in bold. The GDNF precursor protein is processed to a mature secreted form that exists as a homodimer. Each GDNF monomer contains seven conserved cysteine residues, including Cys-101, which is used for inter-chain disulfide bridging, and others that are involved in the intramolecular ring formation known as the cysteine-knot configuration.

The human GDNF polypeptide may be a recombinant human GDNF polypeptide. The term "recombinant" when made in reference to a protein or a polypeptide refers to a protein or polypeptide molecule that is not isolated from a natural source *(e.g.,* biological sample), *e.g.,* which is expressed from a recombinant nucleic acid construct created by means of molecular biological techniques. Referring to a nucleic acid construct as "recombinant" therefore indicates that the nucleic acid molecule has been manipulated using genetic engineering, *i.e.* by human intervention. Recombinant nucleic acid constructs may for example be introduced into a host cell by transformation (e.g., transduction or transfection).

Functional variants or fragments of native mature human GDNF protein may include one or more amino acid substitutions, deletions and/or additions relative to the native mature human GDNF protein, and may have a biological activity that is lower, equivalent or higher than that of the native mature human GDNF protein. For example, the functional variant or fragment has an activity that is equivalent (*e.g.,* between 90% to 110%) or higher (*e.g.,* more than 110%) to that of the native mature human GDNF protein. The variant may comprise one or more conservative substitutions. Conservative amino acid substitutions are known in the art, and include amino acid substitutions in which one amino acid having certain physical and or chemical properties is exchanged for another amino acid that has the same chemical or physical properties. For instance, the conservative amino acid substitution can be an acidic amino acid substituted for another acidic amino acid (*e.g.,* Asp to Glu or vice-versa), an amino acid with a nonpolar side chain substituted for another amino acid with a nonpolar side chain (e.g., Ala, Gly, Val, Ile, Leu, Met, Phe, Pro, Trp, Val, etc.), a basic amino acid substituted for another basic amino acid (Lys, Arg, etc.), an amino acid with a polar side chain substituted for another amino acid with a polar side chain (Asn, Cys, Gln, Ser, Thr, Tyr, etc.). The variants can comprise the amino acid sequence of the native GDNF protein or polypeptide with at least one non-conservative amino acid substitution. Preferably, the non-conservative amino acid substitution(s) enhance(s) the activity of the variant relative to that of the native mature human GDNF protein. The human GDNF polypeptide may have the ability to bind to the RET receptor. The human GDNF polypeptide may have the ability to bind to the NCAM receptor.

For example, the human GDNF polypeptide comprises at least 10, 15 or 20 amino acids (*e.g.,* contiguous amino acids) from the mature human native GDNF protein. The human GDNF polypeptide may comprise the sequence ETTYDKILKNLSRNR (gliafin, SEQ ID NO:3), which corresponds to residues 153-167 of SEQ ID NO: 1 and is the putative binding domain of human GDNF to the NCAM receptor (see, Nielsen et al., J Neurosci. 2009 Sep 9; 29(36): 11360-11376). Alternatively, the human GDNF polypeptide comprises at least 25, 30, 35, 40, 45, 50, 60, 70, 80, 90 or 100 amino acids (*e.g.,* contiguous amino acids) from the mature human native GDNF protein.

For example, the human GDNF polypeptide comprises an amino acid sequence that is at least 50%, 60% or 70% identical to the sequence of residues 78-211 depicted in **FIG. 178A** (SEQ ID NO:1). Or the human GDNF polypeptide comprises an amino acid sequence that is at least 80% identical to the sequence of residues 78-211 depicted in **FIG. 17A** (SEQ ID NO:1). Or the human GDNF polypeptide comprises an amino acid sequence that is at least 85% identical to the sequence of residues 78-211 depicted in **FIG. 17A** (SEQ ID NO:1). Or the human GDNF polypeptide comprises an amino acid sequence that is at least 90% identical to the sequence of residues 78-211 depicted in **FIG. 17A** (SEQ ID NO:1). Or the human GDNF polypeptide comprises an amino acid sequence that is at least 95% identical to the sequence of residues 78-211 depicted in **FIG. 17A** (SEQ ID NO:1). Or the human GDNF polypeptide comprises an amino acid sequence that is at least 98% identical to the sequence of residues 78-211 depicted in FIG. 17A (SEQ ID NO:1). Or the human GDNF polypeptide comprises an amino acid sequence that is at least 99% identical to the sequence of residues 78-211 depicted in **FIG. 17A** (SEQ ID NO:1). Or the human GDNF polypeptide comprises or consists of the sequence of residues 78-211 depicted in **FIG. 17A** (SEQ ID NO:1). "Identity" refers to sequence identity between two polypeptides. Identity can be determined by comparing each position in the aligned sequences. Methods of determining percent identity are known in the art, and several tools and programs are available to align amino acid sequences and determine a percentage of identity including EMBOSS Needle, ClustalW, SIM, DIALIGN, etc. As used herein, a given percentage of identity with respect to a specified subject sequence, or a specified portion thereof, may be defined as the percentage of amino acids in the candidate derivative sequence identical with the amino acids in the subject sequence (or specified portion thereof), after aligning the sequences and introducing gaps, if necessary to achieve the maximum percent sequence identity, as generated by the Smith Waterman algorithm (Smith & Waterman, J. Mol. Biol. 147: 195-7 (1981)) using the BLOSUM substitution matrices (Henikoff & Henikoff, Proc. Natl. Acad. Sci. USA 89:10915-9 (1992)) as similarity measures. A "% identity value" is determined by the number of matching identical amino acids divided by the sequence length for which the percent identity is being reported.

Covalent modifications of the human GDNF polypeptide are included within the scope of this disclosure. For example, the native glycosylation pattern of the human GDNF polypeptide may be modified (Beck et al., Curr. Pharm. Biotechnol. 9: 482-501, 2008; Walsh, Drug Discov. Today 15: 773-780, 2010), and linking the human GDNF polypeptide to one of a variety of nonproteinaceous polymers, *e.g.,* polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337. The human GDNF polypeptide may comprise one or more modifications that confer additional biological properties to the polypeptide such as protease resistance, plasma protein binding, increased plasma half-life, tissue or intracellular penetration, etc. Such modifications include, for example, covalent attachment of molecules/moiety to the polypeptide such as fatty acids (*e.g.,* C₆-C₁₈), attachment of proteins such as albumin (see, *e.g.,* U.S. Patent No. 7,268,113); sugars/polysaccharides (glycosylation), biotinylation or PEGylation (see, *e.g.,* U.S. Patent Nos. 7,256,258 and 6,528,485). The human GDNF polypeptide may also be conjugated to moieties to induce its multimerization or oligomerization (*e.g.,* tetramerization), for example by fusing the human GDNF polypeptide to an oligomerization domain or to a molecule that may be oligomerized (*e.g.,* biotin that may bind to 4 binding sites on streptavidin). The human GDNF polypeptide may also be conjugated to moieties that will target the GDNF polypeptide to the distal colon or to specific cells of the distal colon (*e.g.,* Schwann cells and/or precursor thereof), for example using an antibody, antibody fragment or ligand that binds to a marker present on cells from the distal colon.

The human GDNF polypeptide can also be conjugated to one or more therapeutic or active agents (*e.g*., to a drug, or to another polypeptide to form a fusion polypeptide). Any method known in the art for conjugating the human GDNF polypeptide to another moiety *(*e.g., active agent) may be employed, including those methods described by Hunter et al. (1962) Nature, 144:945; David et al. (1974) Biochemistry, 13: 1014; Pain et al. (1981) J. Immunol. Meth., 40:219; Nygren, J. Histochem. and Cytochem., 30:407 (1982), and Hermanson, Bioconjugate Techniques 1996, Academic Press, Inc., San Diego.

The effective dose of recombinant GDNF polypeptide administered or for administration to the human subject may correspond to a dose of about 5 µg to about 20 µg in a mouse pup, which is the range shown to be effective in the studies described herein. A 10 µl enema comprising a recombinant GDNF solution was administered to mouse pups. 10 µl is estimated to correspond to the volume necessary to fill the distal colon and rectum of the pups. Accordingly, administration of 5 µg GDNF in mice (pups) is achieved by administering 10 µl of a 0.5 µg/µl GDNF solution, and administration of 20 µg GDNF in mice (pups) is achieved by administering 10 µl of a 2.0 µg/µl GDNF solution. The volume required to fill the distal colon and rectum of a human baby may be estimated using the formula: 10ml x weight of the baby (in kg). Accordingly, a dose of 5 µg GDNF in mice corresponds to about 5 mg per kg in a human baby, and a dose of 20 µg GDNF in mice corresponds to about 20 mg per kg in a human baby. Thus, the effective dose of recombinant GDNF polypeptide administered or for administration to the human subject is about 5 mg to about 20 mg per kg, preferably about 10 mg to about 15 mg per kg. The recombinant GDNF polypeptide may be administered or is for administration through a 0.5 mg/ml to 2 mg/ml composition (solution or gel).

Formulations for rectal/distal colon administration may be presented as a suppository, which may be prepared by mixing a composition described herein with one or more suitable nonirritating carriers comprising, for example, cocoa butter, polyethylene glycol, a suppository wax, or a salicylate, and which is solid at room temperature, but liquid at body temperature and, therefore, will melt in the appropriate body cavity and release the composition comprising GDNF. More recently, liquid suppositories have been developed. Liquid suppositories typically contain thermosensitive and/or mucoadhesive polymers such as poloxamers, Carbopol^{®} (crosslinked polyacrylic acid polymers), sodium alginate, polycarbophil, hydroxypropyl methylcellulose (HPMC), hydroxyethyl cellulose, and methylcellulose.

Formulations for rectal/distal colon administration may also be presented as an enema, a liquid-drug solution, suspension or emulsion that is injected into the rectum and the distal colon. The liquid in which the GDNF polypeptide is diluted may be water or a saline solution, for example.

Formulations for rectal/distal colon administration may also be in the form of a rectal foam or gel. Rectal gels are semi-solid formulations that contain a solvent trapped within a polymer network to create a viscous consistency. Viscosity of the gel can be modified by the addition of co-solvents (*e.g.,* glycerin and propylene glycol) and electrolytes. Foams comprise a hydrophilic liquid continuous phase containing a foaming agent and a gaseous dispersion phase distributed throughout. Following rectal administration, they transition from a foam state to a liquid or semi-solid state on the mucosal surface. Foaming agents are typically amphiphilic substances that are important for foam generation and stabilization. The molecules contain hydrophilic components that are soluble in the aqueous phase and hydrophobic components that form micelles to minimize contact with the aqueous phase.

Administration into the rectum/distal colon may be performed using currently available endoscopes or specialized catheters designed for rectal administration or injection into the distal colon wall of medications and liquids, which may be placed safely and remain comfortably in the rectum for repeated use.

The composition comprising GDNF polypeptide may be administered according to any suitable dosage regimen, for example four times-a-day, twice-a-day, once-a-day, twice-a-week, once-a-week, etc. The treatment may be performed for any suitable period of time to achieve the desired effect, for example for 1 week, 2 weeks, 3 weeks or more.

The above-mentioned treatment may comprise the use/administration of more than one (*i.e.* a combination of) active/therapeutic agent, one of which being the above-mentioned pharmaceutical composition comprising a GDNF polypeptide. The combination of therapeutic agents and/or compositions may be administered or co-administered (*e.g.,* consecutively, simultaneously, at different times) in any conventional dosage form. Co-administration as used herein refers to the use of more than one therapy in the course of a coordinated treatment to achieve an improved clinical outcome. The pharmaceutical composition comprising a GDNF polypeptide described herein may be used in combination with other therapies or drugs, for example analgesics or anti-inflammatory agents. The pharmaceutical composition comprising a GDNF polypeptide described herein may also be used in combination with an agent that stimulate ENS progenitor proliferation, such as a neurotrophic molecule.

The above-mentioned treatment with a composition comprising GDNF polypeptide may be performed in combination with surgery (e.g., pull-through surgery of the Swenson, Soave or Duhamel type). Especially for neonates with HSCR, clinicians often recommend a trial of daily enema treatments prior to surgery. Addition of recombinant GDNF to the enema might increase the likelihood that children with HSCR responded well to pre-operative enema therapy. Accordingly, the above-mentioned treatment with a composition comprising GDNF polypeptide may be performed prior to pull-through surgery. Saline enemas are also commonly used in children with HSCR after pull-through surgery. Post-surgical problems in HSCR patients are believed to be due at least in part to hypoganglionosis in retained distal bowel, the so-called "transition zone". Thus, addition of recombinant GDNF to the enema may be useful to correct the hypoganglionosis in retained distal bowel after surgery. Accordingly, the above-mentioned treatment with a composition comprising GDNF polypeptide may be performed after pull-through surgery.

The above-mentioned treatment with a composition comprising GDNF polypeptide may be performed in combination with ENS stem cell-based therapies, which are being considered for the treatment of HSCR. GDNF may be a useful adjunct to these therapies to promote engraftment.

HSCR is clinically subdivided into short-segment (S-HSCR) and long-segment forms (L-HSCR). S-HSCR, which occurs in >80% of cases, means the ENS is absent from rectum and sigmoid colon. L-HSCR means longer regions of distal bowel are aganglionic. The method/use described herein is for the treatment of a human patient suffering from S-HSCR or L-HSCR. Thus, the method/use described herein may be for the treatment of a human patient suffering from S-HSCR. Alternatively, the method/use described herein may be for the treatment of a human patient suffering from L-HSCR.

The HSCR patient may be an adult patient or pediatric patient. Thus, for example, the HSCR patient is a pediatric patient, preferably a patient that is less than 5, 4, 3 or 2 year-old, more preferably a patient that is less than 1 year-old or less than 6 month-old. In particular, the patient is a male.

HSCR has been associated with mutations in *RET, EDNRB, SOX10, PHOX2B,* and *ZFHX1B,* as well as with Down syndrome or Trisomy 21 (Collagen Vl-associated HSCR). There is also a significant sex difference with male to female ratio as high as 5 to 1. The HSCR may be Collagen Vl-associated HSCR. The HSCR may be *EDNRB* mutation-associated HSCR. The HSCR may be male-biased HSCR. The HSCR may be a *RET* mutation-associated HSCR, *e.g.,* HSCR associated with a mutation that reduces *RET* expression and/or activity in cells from the distal colon. The mutation may be a mutation in *RET* or in a protein involved in *RET* signaling. The mutation may be a mutation in the RET protein. Mutations in the RET gene on chromosome 10q11.2 have been shown to account for 50% of familial and 15-20% of sporadic cases of HSCR, most of which (~75%) were associated with L-HSCR.

Alternatively, the HSCR is not a *RET* mutation-associated HSCR.

### MODE(S) FOR CARRYING OUT THE INVENTION

The present invention is illustrated in further details by the following non-limiting examples.

### Example 1: Materials and Methods

***Mice.** Holstein (Tg[Sox3-GFP, Tyr]HolNpIn*), *TashT* (*Tg[SRY-YFP, Tyr]TashTNpIn*), and *G4-RFP (Gata4p[5kb]-RFP)* lines were as previously described (all maintained on a FVB/N genetic background) (39, 40, 54), whereas *Piebald-lethal* (*Ednrb*^{s-l}; JAX stock # 000308; C3H/HeJ-C57BL/6 mixed background) and *Dhh-Cre* (*Tg[Dhh-cre]1Mejr*; JAX stock # 012929; FVB/N background) were obtained from The Jackson Laboratory. Other mouse lines used were *R26^{[Floxed Stop]YFP}* (*Gt[ROSA]26Sor^{tm1(EYFP)Cos}*; provided by F. Costantini (Columbia University, USA) and maintained on an FVB/N background) (80).

For all enema treatments, mutant mouse pups were identified at P3 via pigmentation-based genotyping. Unless specified otherwise (see **FIG. 1****),** 10 µl enemas consisting of a 1 µg/µl solution of recombinant human mature GDNF (Peprotech cat. # 450-10) diluted in PBS were administered daily between P4 to P8. Clinical grade GDNF (Medgenesis Therapeutix Inc., Canada) and a previously described 6XHis-tagged version (53) used for some experiments had similar efficiency. Other tested molecules (1µg/µl solution in 10µl enemas) included the serotonin receptor (5-HT4R) agonist RS67506 (R&D Systems, Cat. # 0990), Noggin (Sigma, Cat. # SRP4675), endothelin-3 (Sigma, Cat. # E9137), serotonin (Sigma, Cat. # H9523), and L-ascorbic acid (Sigma, Cat. # A4403). Enemas were administered using a 24-gauge gavage needle (Fine Science Tools, Canada) attached to a micropipette. The head of the gavage needle was introduced in the rectum just beyond the anus (pre-lubricated with Vaseline^{™}), and enemas were injected over the course of a few seconds. Pups were then placed back with their mother, and either sacrificed at P20 for tissue analysis or checked daily to track survival.

For EdU incorporation assays, mouse pups received 10 µl intraperitoneal injections of a 10mM EdU solution (ThermoFisher Scientific, Cat. # C10337) once a day during the 5-day (P4 to P8) GDNF enema treatment.

***Tissue processing.*** All bowel tissues to be labelled were cut longitudinally along the mesentery, washed in PBS, pinned down on Sylgard-coated petri dishes, fixed with 4% PFA at 4°C overnight, and finally microdissected to separate longitudinal/circular muscles (containing myenteric neural ganglia) from the submucosa/mucosa layer (containing submucosal neural ganglia). For experiments involving *ex vivo* analyses of living tissues, longitudinal/circular muscles and submucosa/mucosa layers were similarly microdissected, but without fixation. Instead, this procedure was performed in ice-cold oxygenated Krebs solution (NaH₂PO₄-2H₂O 0.187 g/L, NaCl 6.84 g/L, KCI 0.35 g/L, NaHCO₃ 2.10 g/L, Glucose 1.98 g/L, CaCl₂-2H₂O 0.368 g/L, MgCl₂-6H₂O 0.244 g/L). For histological analyses, PFA-fixed bowel tissues were prepared as described above but without further microdissection, and fixed full-thickness bowel segments were then embedded in paraffin and transversally sectioned at 10 µm.

***Tissue labelling and imaging.*** For immunofluorescence staining, whole microdissected tissues were permeabilized for 2 hours in blocking solution (10% FBS and 1% Triton^{™} X-100, in PBS) before being sequentially incubated with specific primary (at 4°C overnight) and relevant secondary (at room temperature for 2 hours) antibodies, both diluted in blocking solution that was also used to wash tissues between all steps. All antibodies and dilution factors are listed in Table 1. EdU was detected using the Invitrogen Click-iT EdU Imaging Kit (ThermoFisher Scientific, Cat. # C10337) in accordance with the manufacturer's instructions. For histological analyses, cross-sections of full-thickness bowel tissues were stained with hematoxylin and eosin (H&E) as previously described (83).

**Table 1: List of primary antibodies and dilution factors used for immunofluorescence**

| **Antibody** | **Source** | **Catalog number** | **RRID reference** | **Dilution** |
|---|---|---|---|---|
| 6X His | R&D systems | MAB050 | AB_357353 | 1:500 |
| CalR | Swant | CG1 | AB_10000342 | 1:500 |
| ChAT | Millipore | AB144P | AB_2079751 | 1:100 |
| GFP | Abcam | Ab290 | AB_303395 | 1:500 |
| HuC/D | Molecular Probes | A-21271 | AB_221448 | 1:500 |
| HuC/D (ANNA-1) | Gift from Vanda Lennon | --- | AB_2313944 | 1:2000 |
| Ki67 | Abcam | ab15580 | AB_443209 | 1:500 |
| NCAM | Abcam | ab5032 | AB_2291692 | 1:500 |
| NOS1 | Santa Cruz Biotechnology | sc-648 | AB_630935 | 1:200 |
| RET | R&D systems | MAB718 | AB_2232594 | 1:500 |
| SOX10 | Santa Cruz Biotechnology | sc-17342 | AB_2195374 | 1:200 |
| SubP | Abcam | ab67006 | AB_1143173 | 1:500 |
| TH | Abcam | ab137869 | --- | 1:500 |
| TUJ1 | Covance | MRB-435P | AB_663339 | 1:1000 |
| VIP | Abcam | ab8556 | AB_306628 | 1:500 |

All immunofluorescence images were acquired with either a 20X or a 60X objective on a confocal microscope (either Nikon A1R or Zeiss 710), except for H&E-stained sections that were imaged with a 10X objective using an Infinity-2 camera (Lumenera Corporation) mounted on a Leica DM 2000 microscope (Leica Microsystems Canada). Image analysis was performed with ImageJ, using the "multi-point" function for cell counting, and the "polygon selection" function for calculation of surface area.

***In vivo and ex vivo analysis of colonic motility.** In vivo* analysis of distal colonic motility in P20 mice was performed using the bead latency test. Mice were anesthetized with 2% isoflurane and a 2 mm glass bead (Sigma, Cat. # 1.04014) was inserted into the distal colon with a probe over a distance of 0.5 cm from the anus. Each mouse was then isolated in its cage without access to food and water, and monitored for the time required to expel the glass bead, which was taken as a proxy for distal colonic transit. The maximal time allowed to expel the bead was 30 minutes.

For *ex vivo* analysis of colonic motility, strips of living muscles from most distal colon (1cm from the anus) of P20 mice were prepared as described above, and attached in the longitudinal direction in a Schuler organ bath (Harvard apparatus) filled with oxygenated Krebs solution. Muscle strips were initially stretched with a preload of 2 g of tension for 60 min, and contraction/relaxation of longitudinal muscles was then continuously recorded with a myograph (Narco Biosystems Inc., Model F-60) coupled to a computer equipped with the BIOPAC student Lab 4.0.2 software (BIOPAC Systems Inc.). Electrical field stimulation (EFS) was applied with a voltage stimulator (BIOPAC Systems Inc., Model BSL MP36/35) connected to electrodes, using parameters that activate enteric neurons without directly activating muscles (12 V, 20 Hz, 10s train duration, and 300 µs stimulus pulse duration). This procedure was repeated 3 times, with 10 min washout periods between stimulations. To characterize the nitrergic and cholinergic components of EFS-induced contractile responses, N-nitro-L-arginine methyl ester (L-NAME; Sigma, Cat. # N5751) and atropine (Sigma, Cat. # A01132) were added to Krebs solution at a final concentration of 0.5 µM and 1 µM, respectively. The area under the curve (AUC) was measured during each EFS-induced response, and data were expressed in ΔAUC (corresponding to the difference between the AUC measured 20s after stimulation minus the AUC measured 20s before stimulation).

***Ex vivo analysis of paracellular permeability.*** Segments of living mucosa from most distal colon (1cm from the anus) of P20 mice were prepared as described above, and mounted in Ussing chambers with 0.5 cm² exposed surface area (Warner Instruments, Model U-9926). Each chamber contained 5 ml of DMEM/F12 medium (Wisent, Cat. # 319-085-CL), which was maintained at 37°C and continuously oxygenated (95% O₂ / 5% CO₂). After a 30 min equilibration period, 200 µl of apical medium was replaced with 200 µl of a 1 mg/ml solution of fluorescein isothiocyanate-conjugated dextran 4kDa (FD4; Sigma, Cat. # 60842-46-8). Fluorescence intensity of basolateral aliquots of 150 µl, reflecting paracellular transit from the luminal surface, was then measured every 30 min over a period of 3 hours, using a fluorimeter (TECAN, Model Infinite M1000). Fluorescence intensity was finally converted in amount of FD4 by comparison to a standard curve, and the average value for the 3-hour period was used to calculate paracellular permeability, which was expressed in ng of FD4 per surface of mucosa area per min (ng/cm²/min).

***Microbiome analysis.*** Stool isolation, microbiome sequencing and data analysis were performed as previously described (38). Briefly, mice were sacrificed at P20 and their feces were directly collected from the colon (3 fecal pellets per mouse). Bacterial DNA was then extracted using the QIAamp^{®} Fast DNA Stool Mini Kit (QIAGEN, Cat. # 51604), and the V5-V6 region of the 16S rRNA gene was PCR amplified with a collection of previously described barcoded primers (84). Raw sequences generated with an Illumina MiSeq sequencer were paired and processed using the MOTHUR pipeline (85), and the BIOM package (86) was subsequently used to transfer biom files into R (87) for generating graphs of relative taxa abundance and beta diversity.

***Western blot analysis.*** Organs from P8 or P20 mice were weighed and dissolved in RIPA buffer (25 mM Tris-HCL [pH7.6], 150mM Sodium Chloride, 1% Nonidet P-40, 1% Sodium Deoxycholate, 0,1% Sodium Dodecyl Sulfate, containing 1X Roche Complete protease inhibitors), using 1 mL for every 100 mg of tissue. Samples were then sonicated on ice and centrifuged at 14,000 rpm for 15 minutes at 4°C, keeping the supernatants for western blot analysis. Equal volumes of samples were electrophoretically separated in an 18% sodium dodecyl sulfate-polyacrylamide gel (SDS-PAGE) and transferred to Immun-blot^{®} PVDF membranes (Bio-Rad, Cat. # 1620177). Membranes were subsequently incubated in blocking solution (5% skimmed milk and 0.1% Tween^{®} 20, in TBS), followed by incubation with either mouse anti-GDNF (Santa Cruz Biotechnology, Cat. # sc-13147; 1:500 dilution factor) or rabbit anti-αTubulin (Abcam, Cat. # ab176560; 1:70,000 dilution factor) primary antibodies, and then relevant horseradish peroxidase-conjugated secondary antibodies, all diluted in blocking solution. Each incubation was for 60 min at room temperature, each time interspersed by 3 washes with blocking solution. Proteins were finally visualized using Immobilon western chemiluminescent HRP substrate (Millipore Sigma, Cat. # WBKLS0050) and Fusion FX imaging system (Vilber).

***Ex vivo time-lapse imaging and culture of murine aganglionic colon tissues.*** For time-lapse imaging, strips of living muscles from the last cm of distal colon from P4 *Hol*^{*Tg*/*Tg*};*G4-RFP* double transgenic pups were prepared as described above, and pinned down in Sylgard-coated 35 mm ibidi µ-dishes (ibidi, Cat. # 81156). Muscle strips were cultured in suspension in DMEM/F12 medium (Wisent, Cat. # 319-085-CL) supplemented with 10% FBS and 100IU/ml antibiotic-antimycotic with or without 5 µg/ml GDNF under standard culture conditions (37°C, 5% CO₂). After 72h of culture, each petri dish was placed in a microscope incubation chamber (Okolab) for 10 hours under the same culture conditions, and image stacks (250 µm-thick) of RFP-labelled extrinsic nerves and SCPs were acquired every 10 min, using a 20X objective on a Nikon A1R confocal unit as previously described (39).

For *ex vivo* induction of neurogenesis, strips of living muscles from the last cm of distal colon from P4 *Hol*^{*Tg*/*Tg*} pups were prepared and cultured as described above, with the notable exceptions that culture was performed in presence of 0.5 µM EdU and for a more extended period of time. After 96h of culture, tissues were fixed with PFA and processed for immunofluorescence and EdU labelling.

***Culture of human aganglionic colon tissues.*** Human samples of sigmoid colon were obtained from 12 HSCR patients undergoing Swenson-type surgical resection of the aganglionic zone, as confirmed by histopathological analysis. Nine patients (7 boys and 2 girls; aged between 28 and 1638 days at the time of surgery) were recruited at the Centre Hospitalier Universitaire Sainte-Justine (Montreal, Canada) while 3 patients were recruited at the Children's Hospital of Philadelphia (2 boys and 1 girl; aged between 300 and 1177 days at the time of surgery). After the surgery, full-thickness colon tissues were placed in ice-cold Krebs solution or Belzer UW Cold Storage Solution (Bridge to Life Ltd.) and immediately brought to the relevant research laboratory. Strips of living muscles were then prepared as described above and cut in smaller pieces of 0.5 cm X 0.5 cm. At least one of these small pieces was immediately fixed and kept aside for validation of aganglionosis via immunofluorescence, while the others were cultured for 96h as described above for inducing neurogenesis in mouse tissues. Samples from two patients (aged of 86 and 1638 days at the time of surgery) were in addition cultured for 7 days, under the same conditions. At the end of culture period, all tissues were fixed with PFA and processed for immunofluorescence and EdU labelling.

***Profiling of immune cell populations in P20 colon.** Holstein* mice were treated or not with GDNF by enemas (daily administration of 10 µg of GDNF (10 µl at 1 µg/µl) for 5 consecutive days between P4 to P8. At P20, the colon of GDNF-treated *Holstein* and control (untreated *Holstein* and WT) mice was microdissected, cleaned in cold RPMI medium to remove stool, cut into small pieces, mechanically dissociated, and filtrated (40 µm). Dissociated cells were then recovered by centrifugation, resuspended in 1 ml of ice-cold RPMI with 2% FBS, and counted with a hemocytometer. For every experimental and control groups, 1x10⁶ dissociated cells were incubated for 1h on ice with a cocktail of 14 fluorescently labeled antibodies specific for either lymphoid or myeloid cell populations. Quantitative profiling was subsequently performed using an LSR Fortessa cytometer (BD Biosciences) and FlowJo software (FlowJo LLC).

***Statistics.*** All experiments were performed with a minimum of three biological replicates. Where relevant, the exact number of independent replicates (n) and statistical tests used to calculate P values are included in figures and/or legends. P values were determined using GraphPad Prism 6, with the exception of microbiome data that were analyzed with R software (The R Foundation for Statistical Computing, Vienna, Austria).

### Example 2: GDNF enemas rescue aganglionosis in three mouse models of short-segment HSCR

It was first tested if GDNF enemas in young mice could enhance enteric neuron numbers and restore function in distal aganglionic bowel using three mouse models of short-segment HSCR. Selected lines were *Holstein* (*Hol*^{*Tg*/*Tg*}; a model for Trisomy 21 [Collagen VI]-associated HSCR) (39), *TashT* (*TashT*^{*Tg*/*Tg*}; a model for male-biased HSCR) (40), and *Piebald-lethal* (*Ednrb*^{*s- l*//*s-l*}; a model for *EDNRB* mutation-associated HSCR) (41). The enema volume necessary to fill whole colon, concentration of GDNF homodimer (30 kDa), treatment time window, as well as duration and frequency of therapy were first determined with *Hol*^{*Tg*/*Tg*} pups **(****FIGs. 1A-D****).** It was observed that administration of 100 µg of GDNF reduces survival relative to saline control **(****FIG.** 1C), suggesting that such dose is toxic or worsens the disease. Administration of 1 µg or 25 µg of GDNF had no significant effect on survival, and 10 or 15 µg were shown to be optimal to improve survival. Remarkably, the selected treatment (i.e. daily administration of 10 µg GDNF in PBS as 10 µl enemas for 5 consecutive days between postnatal day [P]4 to P8) prevented death by megacolon in about half of *Hol*^{*Tg*/*Tg*} mice at P28, the maximum age of survival for control *Hol*^{*Tg*/*Tg*} mice **(****FIG. 2A****).** Most animals surviving to P28 reached adult age after GDNF treatment and mice evaluated could reproduce (two tested breeding pairs were fertile). The few males that were allowed to survive beyond P56 (the adult reference age) eventually died from megacolon at P68, P97, P101 and P180, while females eventually died from either megacolon at P63 or dystocia at P138 and P250. Importantly, the same GDNF enema treatment also prevented premature death for more than 60% of *Ednrb*^{s-l//s-l} mice **(****FIG. 2B****)** and for all male *TashT*^{*Tg*/*Tg*} pups **(****FIG. 2C****).** Nine GDNF-treated male *TashT*^{*Tg*/*Tg*} mice kept for over a year looked healthy without any sign of tumor or other adverse effects. Because saline enemas reduce enterocolitis in children with HSCR, separate groups of *Hol*^{*Tg*/*Tg*}, *Ednrb*^{*s-l*//*s-l*} and *TashT*^{*Tg*/*Tg*} mice were treated with PBS via enema, but PBS alone did not enhance survival **(****FIG. 2A-C)**Enema treatment of *Hol*^{*Tg*/*Tg*} mice using Noggin homodimer (46 kDa), endothelin-3 monomer (26 kDa), or the serotonin receptor (5-HT4R) agonist RS67506 (454.41 g/mol) in PBS (each at 10 µg in 10 µl PBS) also failed to increase life expectancy **(****FIG. 1E****).** Collectively, these data suggest that GDNF enemas can be beneficial to mice with HSCR-like distal bowel aganglionosis due to *Holstein, TashT,* or *Piebald-lethal* mutations.

Variability in response to GDNF enemas was observed in mutant mouse lines that are clearly GDNF responsive **(****FIGs. 2A-C)**It was tried without success to increase the overall survival rate of GDNF-treated *Hol*^{*Tg*/*Tg*} animals either by replacing standard chow with a gel diet **(****FIG. 1F****)** or by combining GDNF with other molecules that have been shown to stimulate ENS progenitor proliferation like vitamin C (44), serotonin (45) or endothelin-3 (46) **(****FIG. 1G****).** Gel diet extended life expectancy of control *Hol*^{*Tg*/*Tg*} mice by 5 days on average, but did not further increase survival in the GDNF enema group **(****FIG. 1F****).** It is possible that GDNF enema responsiveness depends to some degree on bowel injury or inflammation that also reduces life expectancy in HSCR mouse models without GDNF treatment (47).

To determine how GDNF enemas enhanced survival of HSCR mouse models, the hypothesis that enhanced survival was caused by postnatal enteric neurogenesis in distal colon aganglionic was tested. P20 animals were analyzed because *Hol*^{*Tg*/*Tg*} mice generally reach this stage even without enema treatment **(****FIG. 2A****).** HuC/D⁺ myenteric neurons were abundant in WT distal colon and absent from the last cm of *Hol*^{*Tg*/*Tg*} colon **(****FIG. 2D****).** SOX10⁺ enteric glia were also abundant within WT myenteric ganglia. In contrast, in *Hol*^{*Tg*/*Tg*} distal colon, SOX10⁺ cells were mainly found within thick extrinsic nerve fibers **(****FIG. 2D****)** where SOX10⁺ SCPs reside (29). Remarkably, distal colon from GDNF-treated *Hol*^{*Tg*/*Tg*} animals had numerous HuC/D⁺ neurons and SOX10⁺ glia organized into ganglia between circular and longitudinal smooth muscles **(****FIG. 2D****).** These myenteric ganglia were primarily adjacent to extrinsic nerve fibers with SOX10⁺ cells. Tuj1 labeling further revealed that GDNF-induced neural ganglia formed interconnected networks in both myenteric and submucosal plexuses **(****FIG. 3****).** Quantification of myenteric neuron density in whole colon of *Hol*^{*Tg*/*Tg*} and male *TashT*^{*Tg*/*Tg*} mice showed GDNF effects are most prominent in distal colon (i.e. final 3 cm), with minor effects in proximal colon **(****FIGs. 2D** and **5A-C)**In the mid-colon of GDNF-treated *Hol*^{*Tg*/*Tg*} mice, the increased neuron density **(****FIG. 2D****)** was mainly due to an enlargement of pre-existing myenteric ganglia, as evidenced by a higher proportion of large ganglia (i.e. >50 neurons) **(****FIG. 4A****).** In the most distal colon, where untreated *Hol*^{*Tg*/*Tg*} mice are normally devoid of enteric neurons, GDNF-treated *Hol*^{*Tg*/*Tg*} mice had an average neuron density that was 40% that of WT mice (5.2 ± 2.2 % *vs* 13.1 ± 3.6 %) **(****FIG. 2D****).** Furthermore, while all GDNF-treated *Hol*^{*Tg*/*Tg*} mice had neurons in the distal colon at the time of dissection, some mice had very low neuron density in distal colon (2.3% or less) and these mice often had megacolon **(****FIG. 1H****).** Remarkably, in GDNF-treated *TashT*^{*Tg*/*Tg*} males, neuron density in the most distal colon increased from 36% (4.7 ± 3.8 % *vs* 13.1 ± 3.6 % of neuronal density) to 108% of WT levels (14.2 ± 4.3 % *vs* 13.1 ± 3.6 % of neuronal density) **(****FIG. 4B-C)**suggesting that it is possible in some models to completely restore normal enteric neuron numbers by GDNF enemas.

Importantly, EdU labeling confirmed that GDNF induced proliferation of neuron and glia progenitors during the 5-day treatment from P4 to P8. Immunofluorescent staining of P20 *Hol*^{*Tg*/*Tg*} colon from mice that received daily EdU injections during the GDNF treatment period revealed many EdU⁺ HuC/D⁺ (presumptive neurons) and EdU⁺ SOX10⁺ (presumptive glia or neuron/glia progenitors) in both myenteric and submucosal ganglia **(****FIGs. 2E-F** and **5A-C).** The percentage of EdU⁺ cells was quite variable from ganglion to ganglion **(****FIG. 5C****).** Only a few ganglia were fully populated by EdU⁺ neurons, and such ganglia were always very small (i.e. composed of 3 neurons). Collectively, these results suggest that GDNF enemas induce proliferation of progenitors in distal colon and that some of dividing cells cluster into new ganglia that express neuronal and glial markers.

### Example 3: GDNF-induced ENS is morphologically and functionally similar to WT

Focusing on the *Hol*^{*Tg*/*Tg*} model, it was next determined to what extent GDNF-induced ENS in the distal colon resembles WT at P20. Average neuron-to-glia ratio within GDNF-induced myenteric ganglia (0.77±0.09 neurons/glia) was statistically similar to WT (0.96±0.06 neurons/glia, *P*=0.16) **(****FIG. 6A****).** Relative proportions of major myenteric neuron subtypes, including ChAT⁺ (choline acetyltransferase) and nNOS⁺ (neuronal nitric oxide synthase) neurons, was also very similar to WT **(****FIGs. 6B-C)**Moreover, many other neuronal subtypes were detected including TH⁺ (tyrosine hydroxylase) dopaminergic neurons, CalR⁺ (calretinin) excitatory motor neurons, VIP⁺ (vasoactive intestinal peptide) inhibitory motor neurons, and SubP⁺ (substance P) excitatory motor neurons **(****FIG. 6C****).** Interestingly, in proximal and mid colon of *Hol*^{*Tg*/*Tg*} mice **(****FIGs. 7A-B)**GDNF treatment also corrected the imbalance of nitrergic (increased) and cholinergic (decreased) neuron subtypes that is observed upstream of the aganglionic segment in both HSCR mouse models and human patients (38, 48-51).

To evaluate function of P20 GDNF-induced myenteric ganglia, colonic motility was analyzed *in vivo* using the bead latency test. Control *Hol*^{*Tg*/*Tg*} mice did not expel a glass bead inserted into their rectum during the 30-minute observation period, but a subset of GDNF-treated *Hol*^{*Tg*/*Tg*} did expel the bead in 10-21 min, a bit slower than WT mice (range of 2-8 min) **(****FIG. 6D****).** Analysis of neuron density in these GDNF-treated *Hol*^{*Tg*/*Tg*} mice revealed a robust inverse correlation between time to expel the bead and neuron density in the distal colon **(****FIG. 8A****).** Colon motility was also evaluated *ex vivo* using strips of distal colon *muscularis externa* from P20 animals attached to force transducers in an organ bath. This system allows electric field stimulation-induced contractions of WT colon muscles to be slightly increased by inhibition of nitric oxide synthase with L-NAME (nitro-L-arginine methyl ester), which can then be robustly counteracted by inhibition of cholinergic signaling with atropine (muscarinic receptor antagonist). Similar to *in vivo* data, colon muscle strips from GDNF-treated *Hol*^{*Tg*/*Tg*} mice displayed one of two distinct response patterns. Some GDNF-treated *Hol*^{*Tg*/*Tg*} colon responses were similar to WT (4/7 mice) while others responded similar to untreated *Hol*^{*Tg*/*Tg*} (3/7 mice) **(****FIG. 6E** and **8B****).** Mean area under the curve (ΔAUC) for contractile responses was graphed separately for GDNF-responsive and GDNF-unresponsive *Hol*^{*Tg*/*Tg*} mice because there appear to be two distinct groups **(****FIG. 6E****).** To indirectly test function of P20 GDNF-induced submucosal ganglia, epithelial permeability to small fluorescently labeled dextran molecules (FD4) in Ussing chambers was analyzed. Once more, colonic tissues from GDNF-treated *Hol*^{*Tg*/*Tg*} mice displayed two distinct response types, with mucosal pieces either impermeable to FD4 like WT tissues, or permeable to FD4 like control *Hol*^{*Tg*/*Tg*} tissues from mice that were not GDNF treated **(****FIG. 6F****).**

To complement ENS analyses, other HSCR-associated bowel anomalies were evaluated. *Hol*^{*Tg*/*Tg*} mouse colon had thicker smooth muscles and more neutrophils than WT mice, but GDNF-treated *Hol*^{*Tg*/*Tg*} mouse colon was similar to WT **(****FIGs. 6G-I** and **9A-B).** Similarly, stool microbiome profiling demonstrated dysbiosis in P20 *Hol*^{*Tg*/*Tg*} mouse colon, but average abundance of several bacterial genera in *Hol*^{*Tg*/*Tg*} mouse colon were indistinguishable from WT after GDNF treatment (*e.g., Desulfovibrio, Escherichia, Helicobater, Mucispirillum, Oscillospira, Parabacteroides,* and *Sutterella*) **(****FIG. 6J****).** A notable exception was *Bacteroides* abundance, which was low in *Hol*^{*Tg*/*Tg*} mice and remained low after GDNF treatment. Accordingly, beta diversity analysis suggests microbial community structure is distinct among WT, *Hol*^{*Tg*/*Tg*} and GDNF-treated *Hol*^{*Tg*/*Tg*} mice **(****FIG. 6K****).**

### Example 4: SCPs within extrinsic nerves are a target of GDNF in aganglionic colon

To elucidate how GDNF induces enteric neurogenesis, GDNF distribution in bowel was first evaluated via Western blot, taking advantage of size differences between recombinant (15 kDa) and endogenous (20 kDa, glycosylated) GDNF proteins in SDS-PAGE gels. Consistent with increased epithelial permeability in distal colon of *Hol*^{*Tg*/*Tg*} mice **(****FIG. 6F****),** recombinant GDNF protein was detected in GDNF-treated *Hol*^{*Tg*/*Tg*} distal colon at P8, but not in proximal colon **(****FIG. 10A****).** Surprisingly, while endogenous GDNF is normally only detected in ileum, recombinant GDNF enemas triggered robust increases in endogenous GDNF throughout the colon **(****FIG. 10A****).** Consistent with GDNF's short half-life (between 34-92h) (52), recombinant GDNF was no longer detected in colon nor in any other tissue at P20, supporting the idea that administered GDNF primarily acts during the treatment period **(****FIG. 11****).** To test for possible GDNF auto-regulatory loops and assess precise locations of recombinant GDNF during treatment, GDNF treatment of *Hol*^{*Tg*/*Tg*} mice was repeated, but now with a 6xHis-tagged version of GDNF (53) (_{His}GDNF). Time-course analysis of distal colon 2h after GDNF treatment on P4, P6 and P8 revealed recombinant GDNF accumulated over time in colon submucosa **(****FIG. 10B****),** smooth muscles, and subsets of enteric neurons **(****FIG. 10C** and **12****)** of *Hol*^{*Tg*/*Tg*} mice. Interestingly, levels of the main GDNF receptor RET also increased **(****FIG. 10B** and **12****),** supporting the hypothesis that GDNF-RET auto-regulatory loops are activated in GDNF-treated colon that could enhance RET signaling even if initial RET levels were low (*e.g.*, in patients with mutations that reduce RET levels and/or activity). Remarkably, both _{His}GDNF and RET were detected in induced neurons close to extrinsic nerves of GDNF-treated *Hol*^{*Tg*/*Tg*} mice **(****FIG. 10C****)** and *Ednrb*^{s-l//s-l} mice.

It was next assessed whether nerve-associated Schwann cells could be GDNF-targeted ENS progenitors. To test this hypothesis, response of Schwann cells in extrinsic nerve fibers to GDNF was first assessed, using live explants of distal colon *muscularis externa* from P4 *Hol*^{*Tg*/*Tg*};*G4-RFP* double transgenic pups. In these mice, neural crest derivatives including SCPs are marked by RFP fluorescence (54). Time-lapse imaging of explants after 72h of culture in presence or absence of GDNF (5 µg/ml) suggested GDNF stimulates both migration and proliferation of SCPs along extrinsic nerves **(****FIG. 10D****).** Impact of GDNF on proliferation of these SCPs was confirmed via immunofluorescence after 96h of *ex vivo* culture. Ki67⁺ SOX10⁺ double positive cells within extrinsic nerves tripled upon exposure to GDNF (5.5±0.4% without GDNF vs 15.2±2.1% with GDNF) **(****FIG. 10E, F).** Although some of the GDNF-induced neurons that express RET are derived from Schwann cells, RET is possibly not needed to activate these precursors in aganglionic bowel as GDNF signaling in Schwann cells is instead mediated by NCAM (35). In line with this, the data reported in **FIG. 10I** shows NCAM but not RET expression in Schwann cells of extrinsic nerves in aganglionic mouse colon. These results suggest that GDNF therapy may be used even in patients having reduced RET levels and/or activity, *e.g.*, having mutations in RET or in the RET signaling pathway.

To further demonstrate SCPs are a source of GDNF-induced neurons and glia, *in vivo* genetic cell lineage tracing with the Schwann lineage-specific Cre driver transgene *Dhh-Cre* and the *Rosa26* Cre reporter allele *R26^{[Floxed Stop]YFP}* in the *Holstein* [FVB/N] mutant background was used. Intriguingly, untreated *Dhh-Cre*^{Tg/+};*R26*^{YFP/+} and *Hol*^{*Tg*/*Tg*};*Dhh-Cre*^{Tg/+};*R26*^{YFP/+} animals had a lower than expected number of YFP⁺ SCP-derived neurons and glia in proximal and mid colon. While SCPs were previously reported to contribute 20% of colonic neurons in a mixed C57BL/6-129Sv genetic background at 1 month of age (29), they were found to contribute only 5-7% of myenteric neurons in a pure FVB/N genetic background at P20, and this contribution increased to 10-11% in the presence of the homozygous *Holstein* mutation **(****FIGs. 13A,B)****.** Remarkably, SCP-derived (YFP⁺) cells made up 34% of distal colonic neurons in GDNF-treated *Hol*^{*Tg*/*Tg*};*Dhh-Cre*^{Tg/+};*R26*^{YFP/+} animals **(****FIGs. 10G,H).** By daily EdU administration during GDNF treatment from P4 to P8, four subgroups of induced myenteric neurons were identified based on cellular origin (YFP fluorescence) and/or EdU incorporation **(****FIGs. 10G****,H** and **14C).** While this work confirmed that *Dhh⁺* SCPs are a source of GDNF-induced neurons and glia in both myenteric **(****FIGs. 10G,H)** and submucosal **(****FIG. 13C****)** plexus, it also revealed that a majority of induced neurons (66%) were YFP-negative, suggesting non-SCP origin (i.e. non-Dhh-expressing cell type(s)). Moreover, a majority of induced neurons (62%) did not incorporate EdU, regardless of cellular origin **(****FIGs. 10G,H)**raising the possibility that neurogenesis might result from transdifferentiation (i.e., direct differentiation of a postmitotic cell into another type of specialized cell) instead of requiring proliferating precursor cells.

### Example 5: GDNF can induce new neurons in human aganglionic colon ex vivo

To test if GDNF could induce new enteric neurons in human tissue, an *ex vivo* model was needed. It was discovered that 96 hrs of GDNF treatment *ex vivo* induced neurons in all *Hol*^{*Tg*/*Tg*} distal colon aganglionic tissues, but neurogenesis was much less efficient than *in vivo.* Only 3 to 67 neurons were seen in these 1 cm-long explants **(****FIGs. 14A** and **15A****).** Moreover, induced neurons rarely clustered into ganglia and such ganglia were always very small **(****FIG. 14B****).** In marked contrast to widespread EdU incorporation into SCPs **(****FIG. 14C** and **15****),** EdU incorporation in induced neurons was also minimal (less than 20% of HuC/D⁺ cells in explant cultures) **(****FIGs. 14B,C)**Control aganglionic tissues from P4 *Hol*^{*Tg*/*Tg*} mice cultured without GDNF remained devoid of neurons **(****FIGs.14A****,C** and **15),** supporting the idea that GDNF could induce neurogenesis in aganglionic distal colon *ex vivo,* but less efficiently than occurred with GDNF enemas *in vivo.*

Finally, it was tested if GDNF could induce neurogenesis in aganglionic human colon muscle from children who had Swenson pull-through surgery to resect aganglionic distal bowel. The cohort consisted of 12 children. Epidemiologic characteristics were typical of HSCR (i.e., mostly sporadic, male-biased, short-segment) **(Table 2).** Patients who underwent Soave surgery were not enrolled because *muscularis externa* is not resected from the most distal colon with this approach. For each subject, full-thickness resected aganglionic colon was collected on the day of surgery and immediately micro-dissected to remove mucosal and submucosal layers. Remaining *muscularis externa* was cut into smaller pieces and cultured with or without GDNF. Exposure to GDNF for 96h markedly increased the proportion of EdU⁺ SCPs in 9/9 human tissues where EdU was added to media **(****FIG. 14D,E)**Most importantly, new neurons expressing HuC/D, βIII-Tubulin (Tuj1), RET, PGP9.5 and PHOX2B were also detected in three HSCR explants **(****FIGs. 14F****,G** and **16).** These three explants were from the youngest children of our cohort (28 to 44 days old) **(****FIG. 14G** and **Table 2).** Two of these young children had sporadic HSCR with unknown genetic causes. The third child had a MEN2A syndrome-associated *RET* mutation **(Table 2).** For older children, it was tested whether longer GDNF treatment might be beneficial. Very interestingly, culturing distal colon from older children for 7 days (n=2; aged of 86 and 1638 days at the time of surgery) allowed identification of neurons that incorporated EdU in association with extrinsic nerve fibers **(****FIG. 14H****).** Collectively, this data provides compelling evidence that the observations made in mice may be extended to humans.

**Table 2. Overview of HSCR colon samples used for ex vivo preclinical testing of GDNF therapy.**

| **Age at surgery (days)** | **Genetic status** | **Sex** | **Clinical Classification** | **Extent of aganglionosis (cm)** | **Number of neurons** | **EdU⁺ SCPs (%)** |
|---|---|---|---|---|---|---|
| 28 | Sporadic, unknown mutation | M | Short segment disease | 5 | 31 | 38 |
| 36 | Sporadic, unknown mutation | M | Short segment disease | 25 | 27 | 33 |
| 44 | MEN2a syndrome, *RET* mutation | M | Short segment disease | 6 | 15 | 44 |
| 80 | Sporadic, unknown mutation | M | Short segment disease | 7 | 3 | 34 |
| 85 | Sporadic, unknown mutation | M | Short segment disease | 6 | 1 | 32 |
| 86 | Sporadic, unknown mutation | M | Short segment disease | 7 | 0 | 46 |
| 249 | Mowat-Wilson syndrome, *ZFHX1B* mutation | F | Short segment disease | 30 | 0 | 32 |
| 300 | Data not available | M | Data not available | Data not available | 0 | Not quantified |
| 344 | Sporadic, unknown mutation | M | Short segment disease | 26 | 0 | Not quantified |
| 349 | Sporadic, unknown mutation | M | Short segment disease | 9 | 0 | 43 |
| 1177 | Bardet-Biedl Syndrome, BBS1 mutation | F | Long segment disease | 40 | 0 | Not quantified |
| 1638 | Sporadic, unknown mutation | F | Short segment disease | 8 | 0 | 50 |

### Example 6: Effect of GDNF on immune cell populations in the colon of Holstein mice

Multi-color flow cytometry analysis shows that the abnormal proportions of immune cells observed in the colon of untreated Hol^{Tg/Tg} mice become generally normalized back to WT immune cell proportions upon GDNF treatment. For a majority of markers, density plots shows either a complete (B cells, CD73⁺ T cells, CD44⁺CD62L⁺ T cells, RORy⁺ T cells, CX3CR1⁺MHC-II⁺ macrophages) or at least partial (CD4⁺ T cells, CD39⁺ T cells, CD25⁺ T cells, Ly-6C⁺CD64⁺ monocytes, F4-80⁺MHC-II⁺ macrophages, CD11b⁺CD103⁻ macrophages) rescue. The results are summarized in **Table 3.**

**Table 3. Immune cell proportions in WT mice and Hol^{Tg/Tg} mice treated or not with GDNF**

| Cell type | % in WT | % in *Hol*^{Tg/Tg} mice (- GDNF) | % in *Hol*^{Tg/Tg} mice (+ GDNF) |
|---|---|---|---|
| CD4⁺ T cells | 83.7 | 34.5 | 65.4 |
| CD8⁺ T cells | 8.47 | 18 | 15.6 |
| B cells (CD19⁺) | 84.6 | 6.98 | 84.6 |
| CD39⁺ T cells | 0.19 | 6.47 | 1.66 |
| CD73⁺ T cells | 31.1 | 56.7 | 30.4 |
| CD44⁺CD62L⁺ T cells | 17.6 | 5.30 | 15.9 |
| RORy⁺ Th17 cells | 0.18 | 0.26 | 0.18 |
| CD25⁺ T_{reg} cells | 2.67 | 21 | 7.74 |
| F4-80⁺MHC-II⁺ macrophages | 0.43 | 3.72 | 2 |
| Ly-6C⁺CD64⁺ monocytes | 17 | 78.3 | 38 |
| CD11b⁺CD103⁻ macrophages | 0.67 | 34.1 | 5.16 |
| CX3CR1⁺MHC II⁻ macrophages | 0.34 | 0.53 | 0.31 |

Although the present invention has been described hereinabove by way of specific embodiments thereof, it is defined in the appended claims. In the claims, the word "comprising" is used as an open-ended term, substantially equivalent to the phrase "including, but not limited to". The singular forms "a", "an" and "the" include corresponding plural references unless the context clearly dictates otherwise.

### REFERENCES:

1. J. B. Furness, The enteric nervous system and neurogastroenterology. Nat Rev Gastroenterol Hepatol 9, 286-294 (2012).
2. R. O. Heuckeroth, Hirschsprung disease - integrating basic science and clinical medicine to improve outcomes. Nat Rev Gastroenterol Hepatol 15, 152-167 (2018).
3. M. Rao, M. D. Gershon, Enteric nervous system development: what could possibly go wrong? Nat Rev Neurosci 19, 552-565 (2018).
4. J. Amiel et al., Hirschsprung disease, associated syndromes and genetics: a review. J Med Genet 45, 1-14 (2008).
5. R. O. Heuckeroth, K. H. Schafer, Gene-environment interactions and the enteric nervous system: Neural plasticity and Hirschsprung disease prevention. Dev Biol 417, 188-197 (2016).
6. O. Mwizerwa et al., Gdnf is mitogenic, neurotrophic, and chemoattractive to enteric neural crest cells in the embryonic colon. Dev Dyn 240, 1402-1411 (2011).
7. D. Natarajan, C. Marcos-Gutierrez, V. Pachnis, E. de Graaff, Requirement of signalling by receptor tyrosine kinase RET for the directed migration of enteric nervous system progenitor cells during mammalian embryogenesis. Development 129, 5151-5160 (2002).
8. H. M. Young et al., GDNF is a chemoattractant for enteric neural cells. Dev Biol 229, 503-516 (2001).
9. C. S. Tang et al., Identification of Genes Associated With Hirschsprung Disease, Based on Whole-Genome Sequence Analysis, and Potential Effects on Enteric Nervous System Development. Gastroenterology 155, 1908-1922 e1905 (2018).
10. E. S. Emison et al., Differential contributions of rare and common, coding and noncoding Ret mutations to multifactorial Hirschsprung disease liability. Am J Hum Genet 87, 60-74 (2010).
11. J. M. Tilghman et al., Molecular Genetic Anatomy and Risk Profile of Hirschsprung's Disease. N Engl J Med 380, 1421-1432 (2019).
12. H. Gui et al., Whole exome sequencing coupled with unbiased functional analysis reveals new Hirschsprung disease genes. Genome Biol 18, 48 (2017).
13. N. Nagy, A. M. Goldstein, Enteric nervous system development: A crest cell's journey from neural tube to colon. Semin Cell Dev Biol 66, 94-106 (2017).
14. C. S. Tang et al., Uncovering the genetic lesions underlying the most severe form of Hirschsprung disease by whole-genome sequencing. Eur J Hum Genet 26, 818-826 (2018).
15. F. Friedmacher, P. Puri, Hirschsprung's disease associated with Down syndrome: a meta-analysis of incidence, functional outcomes and mortality. Pediatric surgery international 29, 937-946 (2013).
16. O. Swenson, H. F. Rheinlander, I. Diamond, Hirschsprung's disease; a new concept of the etiology; operative results in 34 patients. N Engl J Med 241, 551-556 (1949).
17. O. Swenson, A. H. Bill, Jr., Resection of rectum and rectosigmoid with preservation of the sphincter for benign spastic lesions producing megacolon; an experimental study. Surgery 24, 212-220 (1948).
18. P. K. Tam, Hirschsprung's disease: A bridge for science and surgery. J Pediatr Surg 51, 18-22 (2016).
19. A. Coe et al., Reoperation for Hirschsprung disease: pathology of the resected problematic distal pull-through. Pediatr Dev Pathol 15, 30-38 (2012).
20. A. Pini Prato et al., Hirschsprung disease: do risk factors of poor surgical outcome exist? J Pediatr Surg 43, 612-619 (2008).
21. A. J. Burns et al., White paper on guidelines concerning enteric nervous system stem cell therapy for enteric neuropathies. Dev Biol 417, 229-251 (2016).
22. C. J. McCann, O. Borrelli, N. Thapar, Stem cell therapy in severe pediatric motility disorders. Current opinion in pharmacology 43, 145-149 (2018).
23. K. F. Bergeron, D. W. Silversides, N. Pilon, The developmental genetics of Hirschsprung's disease. Clin Genet 83, 15-22 (2013).
24. F. Obermayr, R. Hotta, H. Enomoto, H. M. Young, Development and developmental disorders of the enteric nervous system. Nat Rev Gastroenterol Hepatol 10, 43-57 (2013).
25. C. L. Yntema, W. S. Hammond, The origin of intrinsic ganglia of trunk viscera from vagal neural crest in the chick embryo. J Comp Neurol 101, 515-541 (1954).
26. N. R. Chevalier et al., How Tissue Mechanical Properties Affect Enteric Neural Crest Cell Migration. Scientific reports 6, 20927 (2016).
27. R. Hotta, R. B. Anderson, K. Kobayashi, D. F. Newgreen, H. M. Young, Effects of tissue age, presence of neurones and endothelin-3 on the ability of enteric neurone precursors to colonize recipient gut: implications for cell-based therapies. Neurogastroenterol Motil 22, 331-e386 (2010).
28. N. M. Le Douarin, M. A. Teillet, The migration of neural crest cells to the wall of the digestive tract in avian embryo. Journal of embryology and experimental morphology 30, 31-48 (1973).
29. T. Uesaka, M. Nagashimada, H. Enomoto, Neuronal Differentiation in Schwann Cell Lineage Underlies Postnatal Neurogenesis in the Enteric Nervous System. J Neurosci 35, 9879-9888 (2015).
30. Y. Watanabe et al., Morphological investigation of the enteric nervous system in Hirschsprung's disease and hypoganglionosis using whole-mount colon preparation. J Pediatr Surg 34, 445-449 (1999).
31. T. Uesaka, M. Nagashimada, H. Enomoto, GDNF signaling levels control migration and neuronal differentiation of enteric ganglion precursors. J Neurosci 33, 16372-16382 (2013).
32. S. Gianino, J. R. Grider, J. Cresswell, H. Enomoto, R. O. Heuckeroth, GDNF availability determines enteric neuron number by controlling precursor proliferation. Development 130, 2187-2198 (2003).
33. H. Wang et al., The timing and location of glial cell line-derived neurotrophic factor expression determine enteric nervous system structure and function. J Neurosci 30, 1523-1538 (2010).
34. A. Hoke et al., Glial cell line-derived neurotrophic factor alters axon schwann cell units and promotes myelination in unmyelinated nerve fibers. J Neurosci 23, 561-567 (2003).
35. G. Paratcha, F. Ledda, C. F. Ibanez, The neural cell adhesion molecule NCAM is an alternative signaling receptor for GDNF family ligands. Cell 113, 867-879 (2003).
36. D. Sjostrand, C. F. Ibanez, Insights into GFRalpha1 regulation of neural cell adhesion molecule (NCAM) function from structure-function analysis of the NCAM/GFRalpha1 receptor complex. J Biol Chem 283, 13792-13798 (2008).
37. E. Suply, P. de Vries, R. Soret, F. Cossais, M. Neunlist, Butyrate enemas enhance both cholinergic and nitrergic phenotype of myenteric neurons and neuromuscular transmission in newborn rat colon. American journal of physiology. Gastrointestinal and liver physiology 302, G1373-1380 (2012).
38. A. M. Toure, M. Landry, O. Souchkova, S. W. Kembel, N. Pilon, Gut microbiota-mediated Gene-Environment interaction in the TashT mouse model of Hirschsprung disease. Scientific reports 9, 492 (2019).
39. R. Soret et al., A collagen VI-dependent pathogenic mechanism for Hirschsprung's disease. J Clin Invest 125, 4483-4496 (2015).
40. K. F. Bergeron et al., Male-Biased Aganglionic Megacolon in the TashT Mouse Line Due to Perturbation of Silencer Elements in a Large Gene Desert of Chromosome 10. PLoS Genet 11, e1005093 (2015).
41. K. Hosoda et al., Targeted and natural (piebald-lethal) mutations of endothelin-B receptor gene produce megacolon associated with spotted coat color in mice. Cell 79, 1267-1276 (1994).
42. J. I. Lake, O. A. Tusheva, B. L. Graham, R. O. Heuckeroth, Hirschsprung-like disease is exacerbated by reduced de novo GMP synthesis. J Clin Invest 123, 4875-4887 (2013).
43. A. Schuchardt, V. D'Agati, L. Larsson-Blomberg, F. Costantini, V. Pachnis, Defects in the kidney and enteric nervous system of mice lacking the tyrosine kinase receptor Ret. Nature 367, 380-383 (1994).
44. F. Fattahi et al., Deriving human ENS lineages for cell therapy and drug discovery in Hirschsprung disease. Nature, (2016)*.*
45. Z. Li et al., Essential roles of enteric neuronal serotonin in gastrointestinal motility and the development/survival of enteric dopaminergic neurons. J Neurosci 31, 8998-9009 (2011).
46. N. Bondurand, D. Natarajan, A. Barlow, N. Thapar, V. Pachnis, Maintenance of mammalian enteric nervous system progenitors by SOX10 and endothelin 3 signalling. Development 133, 2075-2086 (2006).
47. Z. Cheng et al., Murine model of Hirschsprung-associated enterocolitis. I: phenotypic characterization with development of a histopathologic grading system. J Pediatr Surg 45, 475-482 (2010).
48. L. S. Cheng, D. M. Schwartz, R. Hotta, H. K. Graham, A. M. Goldstein, Bowel dysfunction following pullthrough surgery is associated with an overabundance of nitrergic neurons in Hirschsprung disease. J Pediatr Surg 51, 1834-1838 (2016).
49. D. Coyle, A. M. O'Donnell, J. Gillick, P. Puri, Altered neurotransmitter expression profile in the ganglionic bowel in Hirschsprung's disease. J Pediatr Surg 51, 762-769 (2016).
50. A. M. Toure, B. Charrier, N. Pilon, Male-specific colon motility dysfunction in the TashT mouse line. Neurogastroenterol Motil 28, 1494-1507 (2016).
51. I. Zaitoun et al., Altered neuronal density and neurotransmitter expression in the ganglionated region of Ednrb null mice: implications for Hirschsprung's disease. Neurogastroenterol Motil 25, e233-244 (2013).
52. M. Luz, E. Mohr, H. C. Fibiger, GDNF-induced cerebellar toxicity: A brief review. Neurotoxicology 52, 46-56 (2016).
53. D. J. Creedon et al., Neurturin shares receptors and signal transduction pathways with glial cell line-derived neurotrophic factor in sympathetic neurons. Proc Natl Acad Sci U S A 94, 7018-7023 (1997).
54. N. Pilon, D. Raiwet, R. S. Viger, D. W. Silversides, Novel pre- and post-gastrulation expression of Gata4 within cells of the inner cell mass and migratory neural crest cells. Dev Dyn 237, 1133-1143 (2008).
55. T. Shimotake, S. Go, K. Inoue, H. Tomiyama, N. Iwai, A homozygous missense mutation in the tyrosine E kinase domain of the RET proto-oncogene in an infant with total intestinal aganglionosis. Am J Gastroenterol 96, 1286-1291 (2001).
56. S. Ro, S. J. Hwang, M. Muto, W. K. Jewett, N. J. Spencer, Anatomic modifications in the enteric nervous system of piebald mice and physiological consequences to colonic motor activity. American journal of physiology. Gastrointestinal and liver physiology 290, G710-718 (2006).
57. N. M. Joseph et al., Enteric glia are multipotent in culture but primarily form glia in the adult rodent gut. J Clin Invest 121, 3398-3411 (2011).
58. G. M. Kruger et al., Neural crest stem cells persist in the adult gut but undergo changes in self-renewal, neuronal subtype potential, and factor responsiveness. Neuron 35, 657-669 (2002).
59. C. Laranjeira et al., Glial cells in the mouse enteric nervous system can undergo neurogenesis in response to injury. J Clin Invest 121, 3412-3424 (2011).
60. M. Metzger, C. Caldwell, A. J. Barlow, A. J. Burns, N. Thapar, Enteric nervous system stem cells derived from human gut mucosa for the treatment of aganglionic gut disorders. Gastroenterology 136, 2214-2225 e2211-2213 (2009).
61. M. T. Liu, Y. H. Kuan, J. Wang, R. Hen, M. D. Gershon, 5-HT4 receptor-mediated neuroprotection and neurogenesis in the enteric nervous system of adult mice. J Neurosci 29, 9683-9699 (2009).
62. K. Badizadegan et al., Presence of intramucosal neuroglial cells in normal and aganglionic human colon. American journal of physiology. Gastrointestinal and liver physiology 307, G1002-1012 (2014).
63. D. J. Wilkinson, G. S. Bethell, R. Shukla, S. E. Kenny, D. H. Edgar, Isolation of Enteric Nervous System Progenitor Cells from the Aganglionic Gut of Patients with Hirschsprung's Disease. PLoS One 10, e0125724 (2015).
64. S. Almond, R. M. Lindley, S. E. Kenny, M. G. Connell, D. H. Edgar, Characterisation and transplantation of enteric nervous system progenitor cells. Gut 56, 489-496 (2007).
65. S. Hetz et al., In vivo transplantation of neurosphere-like bodies derived from the human postnatal and adult enteric nervous system: a pilot study. PLoS One 9, e93605 (2014).
66. R. Hotta et al., Transplanted progenitors generate functional enteric neurons in the postnatal colon. J Clin Invest 123, 1182-1191 (2013).
67. J. E. Cooper et al., In Vivo Transplantation of Enteric Neural Crest Cells into Mouse Gut; Engraftment, Functional Integration and Long-Term Safety. PLoS One 11, e0147989 (2016).
68. J. E. Cooper et al., In vivo transplantation of fetal human gut-derived enteric neural crest cells. Neurogastroenterol Motil 29, (2017).
69. R. Hotta et al., Isogenic enteric neural progenitor cells can replace missing neurons and glia in mice with Hirschsprung disease. Neurogastroenterol Motil 28, 498-512 (2016).
70. L. A. Stamp et al., Optogenetic Demonstration of Functional Innervation of Mouse Colon by Neurons Derived From Transplanted Neural Cells. Gastroenterology 152, 1407-1418 (2017).
71. C. J. McCann et al., Transplantation of enteric nervous system stem cells rescues nitric oxide synthase deficient mouse colon. Nature communications 8, 15937 (2017).
72. J. Belkind-Gerson et al., Colitis promotes neuronal differentiation of Sox2+ and PLP1+ enteric cells. Scientific reports 7, 2525 (2017).
73. J. Belkind-Gerson et al., Colitis induces enteric neurogenesis through a 5-HT4-dependent mechanism. Inflamm Bowel Dis 21, 870-878 (2015).
74. M. Meir et al., Neurotrophic factor GDNF regulates intestinal barrier function in inflammatory bowel disease. J Clin Invest 129, 2824-2840 (2019).
75. D. K. Zhang et al., Glial-derived neurotrophic factor regulates intestinal epithelial barrier function and inflammation and is therapeutic for murine colitis. J Pathol 222, 213-222 (2010).
76. A. J. Burns, D. Champeval, N. M. Le Douarin, Sacral neural crest cells colonise aganglionic hindgut in vivo but fail to compensate for lack of enteric ganglia. Dev Biol 219, 30-43 (2000).
77. Y. Watanabe et al., Extrinsic nerve strands in the aganglionic segment of Hirschsprung's disease. J Pediatr Surg 33, 1233-1237 (1998).
78. H. Nakamura, T. Lim, P. Puri, Inflammatory bowel disease in patients with Hirschsprung's disease: a systematic review and meta-analysis. Pediatric surgery international 34, 149-154 (2018).
79. S. J. McKeown, M. Mohsenipour, A. J. Bergner, H. M. Young, L. A. Stamp, Exposure to GDNF Enhances the Ability of Enteric Neural Progenitors to Generate an Enteric Nervous System. Stem Cell Reports 8, 476-488 (2017).
80. S. Srinivas et al., Cre reporter strains produced by targeted insertion of EYFP and ECFP into the ROSA26 locus. BMC Dev Biol 1, 4 (2001).
81. H. Enomoto et al., RET signaling is essential for migration, axonal growth and axon guidance of developing sympathetic neurons. Development 128, 3963-3974 (2001).
82. S. Jain et al., Mice expressing a dominant-negative Ret mutation phenocopy human Hirschsprung disease and delineate a direct role of Ret in spermatogenesis. Development 131, 5503-5513 (2004).
83. A. Boulende Sab et al., An Ebox element in the proximal Gata4 promoter is required for Gata4 expression in vivo. PLoS ONE 6, e29038 (2011).
84. I. Laforest-Lapointe, A. Paquette, C. Messier, S. W. Kembel, Leaf bacterial diversity mediates plant diversity and ecosystem function relationships. Nature 546, 145-147 (2017).
85. P. D. Schloss et al., Introducing mothur: open-source, platform-independent, community-supported software for describing and comparing microbial communities. Appl Environ Microbiol 75, 7537-7541 (2009).
86. D. McDonald et al., The Biological Observation Matrix (BIOM) format or: how I learned to stop worrying and love the ome-ome. Gigascience 1, 7 (2012).
87. R. C. Team, R: A language and environment for statistical computing. R Foundation for Statistical Computing, Vienna, Austria. (2014).

## Claims

1. A pharmaceutical composition comprising a recombinant Glial cell line-Derived Neurotrophic Factor (GDNF) polypeptide and a pharmaceutically acceptable carrier for use in treating an enteric neuropathy in a human subject by inducing enteric neurogenesis in an aganglionic or hypoganglionic segment of the distal colon, wherein the composition is administered into the distal colon of the subject rectally *via* enema at a dose of recombinant GDNF polypeptide of about 10 mg to about 15 mg per kg.

2. The pharmaceutical composition for use according to claim 1, wherein the GDNF polypeptide comprises an amino acid sequence having at least 90% identity with amino acids 78-211 of SEQ ID NO: 1.

3. The pharmaceutical composition for use according to claim 2, wherein the GDNF polypeptide comprises amino acids 78-211 of SEQ ID NO: 1.

4. The pharmaceutical composition for use according to any one of claims 1 to 3, wherein the recombinant GDNF polypeptide is at a concentration of 0.5 mg/ml to 2 mg/ml.

5. The pharmaceutical composition for use according to any one of claims 1 to 4, wherein the pharmaceutically acceptable carrier is a saline solution or a gelling agent.

6. The pharmaceutical composition for use according to any one of claims 1 to 5, wherein the pharmaceutical composition is administered (i) once-a-day up to four times a day; and/or for at least 2 consecutive days.

7. The pharmaceutical composition for use according to any one of claims 1 to 6, wherein the pharmaceutical composition is administered prior to or after surgical removal of the aganglionic or hypoanglionic segment in the subject.

8. The pharmaceutical composition for use according to any one of claims 1 to 7, wherein the enteric neuropathy is intestinal hypoganglionosis.

9. The pharmaceutical composition for use according to any one of claims 1 to 7, wherein the enteric neuropathy is Hirschsprung disease (HSCR).

10. The pharmaceutical composition for use according to claim 9, wherein the subject suffers from short-segment HSCR.

11. The pharmaceutical composition for use according to claim 9 or 10, wherein the HSCR is sporadic HSCR.

12. The pharmaceutical composition for use according to any one of claims 9 to 11, wherein the HSCR is associated with a reduced expression or activity of the RET receptor.

13. The pharmaceutical composition for use according to any one of claims 1 to 12, wherein the pharmaceutical composition (i) corrects the imbalance of nitrergic and cholinergic neuron subtypes located upstream of the aganglionic or hypoganglionic segment; (ii) restores distal colon motility in the subject; and/or (iii) restores the proportions of lymphoid and/or myeloid immune cells in the distal colon of the subject.

14. The pharmaceutical composition for use according to any one of claims 1 to 13, wherein the human subject is less than 5-year-old.

## Patentansprüche

1. Pharmazeutische Zusammensetzung, umfassend ein rekombinantes Polypeptid des von Gliazelllinien abgeleiteten neurotrophen Faktors (GDNF) und einen pharmazeutisch annehmbaren Träger zur Verwendung bei der Behandlung einer enterischen Neuropathie bei einem menschlichen Subjekt durch Induktion der enterischen Neurogenese in einem aganglionären oder hypoganglionären Segment des distalen Kolons, wobei die Zusammensetzung in das distale Kolon des Subjekts rektal über einen Einlauf in einer Dosis des rekombinanten GDNF-Polypeptids von etwa 10 mg bis etwa 15 mg pro kg verabreicht wird.

2. Pharmazeutische Zusammensetzung zur Verwendung nach Anspruch 1, wobei das GDNF-Polypeptid eine Aminosäuresequenz mit mindestens 90 % Identität mit den Aminosäuren 78-211 von SEQ ID NO:1 umfasst.

3. Pharmazeutische Zusammensetzung zur Verwendung nach Anspruch 2, wobei das GDNF-Polypeptid die Aminosäuren 78-211 von SEQ ID NO:1 umfasst.

4. Pharmazeutische Zusammensetzung zur Verwendung nach einem der Ansprüche 1 bis 3, wobei das rekombinante GDNF-Polypeptid in einer Konzentration von 0,5 mg/ml bis 2 mg/ml vorliegt.

5. Pharmazeutische Zusammensetzung zur Verwendung nach einem der Ansprüche 1 bis 4, wobei der pharmazeutisch annehmbare Träger eine Salzlösung oder ein Geliermittel ist.

6. Pharmazeutische Zusammensetzung zur Verwendung nach einem der Ansprüche 1 bis 5, wobei die pharmazeutische Zusammensetzung (i) einmal täglich bis zu viermal täglich und/oder an mindestens zwei aufeinanderfolgenden Tagen verabreicht wird.

7. Pharmazeutische Zusammensetzung zur Verwendung nach einem der Ansprüche 1 bis 6, wobei die pharmazeutische Zusammensetzung vor oder nach einer chirurgischen Entfernung des aganglionären oder hypoganglionären Segments bei dem Patienten verabreicht wird.

8. Pharmazeutische Zusammensetzung zur Verwendung nach einem der Ansprüche 1 bis 7, wobei die enterische Neuropathie eine intestinale Hypoganglionose ist.

9. Pharmazeutische Zusammensetzung zur Verwendung nach einem der Ansprüche 1 bis 7, wobei die enterische Neuropathie die Hirschsprung-Krankheit (HSCR) ist.

10. Pharmazeutische Zusammensetzung zur Verwendung nach Anspruch 9, wobei das Subjekt an Kurzsegment-HSCR leidet.

11. Pharmazeutische Zusammensetzung zur Verwendung nach Anspruch 9 oder 10, wobei die HSCR eine sporadische HSCR ist.

12. Pharmazeutische Zusammensetzung zur Verwendung nach einem der Ansprüche 9 bis 11, wobei die HSCR mit einer verminderten Expression oder Aktivität des RET-Rezeptors verbunden ist.

13. Pharmazeutische Zusammensetzung zur Verwendung nach einem der Ansprüche 1 bis 12, wobei die pharmazeutische Zusammensetzung (i) das Ungleichgewicht von nitrergen und cholinergen Neuronsubtypen, die stromaufwärts des aganglionären oder hypoganglionären Segments lokalisiert sind, korrigiert; (ii) die distale Kolonmotilität in dem Subjekt wiederherstellt; und/oder (iii) die Proportionen von lymphoiden und/oder myeloiden Immunzellen in dem distalen Kolon des Subjekts wiederherstellt.

14. Pharmazeutische Zusammensetzung zur Verwendung nach einem der Ansprüche 1 bis 13, wobei das menschliche Subjekt weniger als 5 Jahre alt ist.

## Revendications

1. Composition pharmaceutique comprenant un polypeptide recombinant de facteur neurotrophe dérivé de la lignée cellulaire gliale (GDNF) et un support pharmaceutiquement acceptable pour une utilisation dans le traitement d'une neuropathie entérique chez un sujet humain par induction d'une neurogenèse entérique dans un segment aganglionnaire ou hypoganglionnaire du côlon distal, dans laquelle la composition est administrée dans le côlon distal du sujet rectalement par l'intermédiaire d'un lavement à une dose de polypeptide GDNF recombinant d'environ 10 mg à environ 15 mg par kg.

2. Composition pharmaceutique pour une utilisation selon la revendication 1, dans laquelle le polypeptide GDNF comprend une séquence d'acides aminés ayant au moins 90 % d'identité avec les acides aminés 78-211 de SEQ ID N° : 1.

3. Composition pharmaceutique pour une utilisation selon la revendication 2, dans laquelle le polypeptide GDNF comprend des acides aminés 78-211 de SEQ ID N° : 1.

4. Composition pharmaceutique pour une utilisation selon l'une quelconque des revendications 1 à 3, dans laquelle le polypeptide GDNF recombinant est à une concentration de 0,5 mg/ml à 2 mg/ml.

5. Composition pharmaceutique pour une utilisation selon l'une quelconque des revendications 1 à 4, dans laquelle le support pharmaceutiquement acceptable est une solution saline ou un agent gélifiant.

6. Composition pharmaceutique pour une utilisation selon l'une quelconque des revendications 1 à 5, dans laquelle la composition pharmaceutique est administrée (i) d'une fois par jour à quatre fois par jour ; et/ou pendant au moins 2 jours consécutifs.

7. Composition pharmaceutique pour une utilisation selon l'une quelconque des revendications 1 à 6, dans laquelle la composition pharmaceutique est administrée avant ou après le retrait chirurgical du segment aganglionnaire ou hypoganglionnaire chez le sujet.

8. Composition pharmaceutique pour une utilisation selon l'une quelconque des revendications 1 à 7, dans laquelle la neuropathie entérale est une hypoganglionose intestinale.

9. Composition pharmaceutique pour une utilisation selon l'une quelconque des revendications 1 à 7, dans laquelle la neuropathie entérique est la maladie de Hirschsprung (HSCR).

10. Composition pharmaceutique pour une utilisation selon la revendication 9, dans laquelle le sujet souffre de la HSCR court-segment.

11. Composition pharmaceutique pour une utilisation selon la revendication 9 ou 10, dans laquelle la HSCR est une HSCR sporadique.

12. Composition pharmaceutique pour une utilisation selon l'une quelconque des revendications 9 à 11, dans laquelle la HSCR est associée à une expression ou une activité réduite du récepteur RET.

13. Composition pharmaceutique pour une utilisation selon l'une quelconque des revendications 1 à 12, dans laquelle la composition pharmaceutique (i) corrige le déséquilibre des sous-types de neurones nitrergiques et cholinergiques situés en amont du segment aganglionnaire ou hypoganglionnaire ; (ii) restaure la motilité du côlon distal chez le sujet ; et/ou restaure les proportions de cellules immunitaires lymphoïdes et/ou myéloïdes dans le côlon distal du sujet.

14. Composition pharmaceutique pour une utilisation selon l'une quelconque des revendications 1 à 13, dans laquelle le sujet humain a moins de 5 ans.
